(19) Europäisches Patentamt
European Patent Office
Office européen des brevets

(11) Publication number: 0 046 270
B1

# (12) EUROPEAN PATENT SPECIFICATION

(45) Date of publication of patent specification: 21.05.86

(21) Application number: 81106277.7

(22) Date of filing: 12.08.81

(51) Int. Cl.$^4$: C 07 C 103/78, C 07 C 93/26, C 07 C 69/24, C 07 C 153/07, C 07 C 69/28 // C07C125/065

## (54) Novel derivatives of bio-affecting phenolic compounds and pharmaceutical composition containing them.

(30) Priority: 13.08.80 US 177825

(43) Date of publication of application:
24.02.82 Bulletin 82/08

(45) Publication of the grant of the patent:
21.05.86 Bulletin 86/21

(84) Designated Contracting States:
AT BE CH DE FR GB IT LI LU NL SE

(56) References cited:
DE-A-2 228 670

TETRAHEDRON LETTERS, no. 48, November 1972, Pergamon Press OXFORD (GB) P.C. OELE et al.: "Thermolytic alpha - Elimination of Acetic Acid from Methylacetate Derivatives" pages 4941-4944

(73) Proprietor: INTERx RESEARCH CORPORATION
2201 West 21st Street
Lawrence Kansas 66044 (US)

(72) Inventor: Bodor, Nicholas S.
315 Southwest 91st Street
Gainsville Florida 32608 (US)
Inventor: Sloan, Kenneth B.
1522 Northwest 52nd Terrace
Gainesville, Florida 32605 (US)
Inventor: Pogany, Stefano A.
520 Louisiana Street
Lawrence Kansas 66044 (US)

(74) Representative: Abitz, Walter, Dr.-Ing. et al
Abitz, Morf, Gritschneder P.O. Box 86 01 09
D-8000 München 86 (DE)

(56) References cited:
CHEMICAL ABSTRACTS, vol. 83, no. 23, December 8, 1975, page 361 American Chemical Society abstract no. 192167t COLUMBUS, Ohio (US) R.A. McCLELLAND: "Mechanisms of Acylal Hydrolysis in Sulfuric Acid Solutions"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

The present invention relates to novel, transient prodrug derivatives of biologically active, non-steroidal nuclear monohydroxy or nuclear polyhydroxy benzenoids, and, more especially, relates to certain acyl-X-methyl ether latentiated forms of such compounds, e.g., of the nuclear monohydroxy and trihydroxy sympathomimetic amines.

As employed in this application, the expression "prodrug" denotes a derivative of a known and proven prior art compound, which derivative, when absorbed by warm-blooded animal, "cleaves" in such a manner as to release the proven prior art compound and permits the same to attain a higher bioavailability level than that which could be obtained if the proven compound, per se, were administered.

Furthermore, also as used in this application, the term "transient" denotes "cleavage" of the compounds of this invention in such a manner that the proven prior art form is released and the remaining "cleaved" moiety is non-toxic and metabolized in such a manner that non-toxic, metabolic products are produced.

It is well known that a wide variety of non-steroidal nuclear monohydroxy or nuclear polyhydroxy benzenoids are biologically active. For example, the nuclear monohydroxy sympathomimetic amines, e.g., metaraminol, phenylephrine hydrochloride, deterenol and the like are useful active agents for the treatment or management of a wide variety of disease states or conditions, e.g., glaucoma, inflammation, itching, asthma, nasal congestion, allergic states, bronchospasm, cardiac dysfuncton, bronchial constriction, peripheral vascular disease, shock, and the like. See generally Cutting's *Handbook of Pharmacology, 41,* "Sympathetic Stimulants or Adrenergic Agents", pp. 436—455, sixth Edition (1979). Other especially noteworthy groups of non-steroidal biologically active phenols include tetracyline and structurally related antibiotics; synthetic estrogens; certain vitamins; tyrosine and its derivatives; derivatives of salicyclic acid and structurally related non-steroidal anti-inflammatory and non-narcotic analgesic agents; derivatives of morphine and structurally related narcotic analgesics and narcotic antagonists; relatively simple one-ring phenols, such as phenol itself, which has anti-microbial properties; hydroxyquinoline and its derivatives (e.g., clamoxyquin and chlorquinaldol), many of which have antiamebic and topical antiseptic properties; and numerous other single and multiple ring systems, the latter being bridged and/or fused, optionally containing one or more heteroatoms in the ring or bridge, which are known to be biologically active.

Nevertheless, it is well known to the art that such phenols, e.g., the nuclear monohydroxy sympathomimetic amines, and the various art-recognized therapeutically active derivatives thereof, are characterized by certain inherent disadvantages, notably serious bioavailability and physiological availability problems upon administration. Such reduced availability can be attributed in part to poor lipid solubility [by reason of the presence of the hydrophilic phenolic hydroxyl groups], and also to metabolic losses during and following conventional administration. Other disadvantages associated with the prior art compounds include instability to both air and light, and same may be subject to chemical attack by many agents that are conventionally used in pharmaceutical preparations, as well as a variety of other unfavorable pharmacodynamic properties. Also, for certain applications, e.g., to elicit a topical anti-inflammatory response, relatively high concentrations of drug are required.

Thus, there exists a clear and present need for novel latentiated forms of the non-steroidal bio-affecting phenols, which derivatives would be conspicuously devoid of those disadvantages and drawbacks that to date have characterized the prior art compounds.

Accordingly, a major object of the present invention is the provision of a novel class of prodrugs of bio-affecting phenolic compounds.

Another object of this invention is the provision of a novel class of prodrugs which are acyl-hetero-alkyl ether derivatives of bio-affecting phenols, which prodrugs are essentially free from the unwanted effects associated with the prior art.

Still another object of the invention is to provide a new and useful class of latentiated derivatives of bio-affecting phenols which is characterized by enhanced stability and solubility, can be administered in standard pharmaceutical formulations to warm-blooded animals to elicit a local, topical or systemic physiological or pharmacological beneficial effect, and which exhibits enhanced bioavailability and physiological availability.

Yet another object is to provide a novel class of prodrugs which will elicit a more effective response, at lower concentrations or dosage levels, than its parent molecules.

Other objects, features and advantages of the invention will be apparent to those skilled in the art from the detailed description of the invention which follows.

According to DE—A 2 228 670 medicines having a carboxylic acid function are derivatized, whilst according to the present invention derivatives of medicines having a phenolic function are formed.

J. Am. chem. Soc., *96* (1974), 6158—6165 discusses the mechanismic aspect of the cleavage of acylals in alcohols and aldehydes. No suggestion can be gathered from said reference to the effect that cleavage of acylals can be utilized for improving the activity of medicinal compounds.

According to this invention, all of the aforenoted objects, features and advantages thereof are provided by the novel compounds which are selected from the group consisting of those having the structural formula (I):

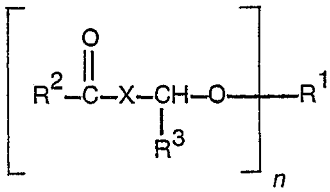 (I)

wherein

X is O, S or $NR^5$ wherein $R^5$ is hydrogen or lower alkyl;

$R^1$ is the nuclear mono- or polydehydroxylated residue of

1. a phenol selected from a group consisting of: hydroxyamphetamine, hydroxyamphetamine-hydrobromide, metaraminol, metaraminol bitartrate, phenylephrine hydrochloride, oxymetazoline hydrochloride, albuterenol (albuterol), carbuterol hydrochloride, deterenol hydrochloride, quinterenol sulfate, soterenol, sulfonterol hydrochloride, isoxuprine hydrochloride (isoxsuprine), nylidrin hydrochloride, bamethan, bamethan sulfate, mesuprine hydrochloride and ritodrine hydrochloride, benserazide, dimethophrine, *dl*-etilefrin, hordenine, p-hydroxyephedrine, ifenprodel, leptodactyline chloride, leptodactyline picrate, norfenefrine, octopamine, phentolamine, pholedrine, synephrine, tyramine, and ethamivan;
2. a phenol which is a salicyclic acid derivative or a structurally related compound selected from a group consisting of: acetaminophen, eugenol, p-aminosalicyclic acid, 4-benezamidosalicyclic acid, calcium benzolypas, biphenamine, buclosamide, homosalate, hydroxyprocaine, methyl salicylate, oxybenzone, dioxybenzone, phenyl aminosalicylate, salicyl alcohol (saligenin), salicylamide, salicylic acid, salicylsalicylic acid (salsalate), sodium salicylate, diflusinal, dibromsalan, fursalan, metabromsalan, bensalan, salethamide, and salcolex, p-aminosalicylic acid hydrazide, ammonium salicylate, 5-bromo-4′-chlorosalicylanilide, 5-bromosalicylhydroxamic acid, p-butylaminosalicylic acid 2-diethylaminoethyl ester, 3′-carboxy-4′-hydroxycinchophen, choline salicylate, 3,5-dibromosalicylaldehyde, fluorosalan, flopropione, gallacetophenone, gentisic acid, 4-hydroxyisophthalic acid, hydroxytetracaine, N-isopropylsalicylamide, niclosamide, osalmid, oxyclozanide, oxyphenbutazone, Pascaine[R] (hydroxyprocaine p-aminosalicylate), phenetsal, phenyl salicylate, resorantel, salacetamide, salicyl, salicylanilide, salazosulfadimidine, salazosulfamide, salicylazosulfapyridine, 4-salicyloxymorphine, 4-sulfanilamidosalicylic acid, o-thymotic acid, 3′,4′,5′-trichlorosalicylanilide, xipamide, amidan, bromosaligenin and 3,5-diiodosalicylic acid;
3. a non-steroidal estrogen phenol selected from a group consisting of: benzestrol, dinestrol, diethylstilbestrol, hexestrol, mestilbol and promethestrol;
4. a phenol of tetracycline-type antibiotics selected from a group consisting of: tetracycline, tetracycline-hydrochloride, chlortetracycline, demeclocycline, doxycycline, oxytetracycline, apicycline, clomocycline, guamecycline, methacycline, rolitetracycline, rolitetracycline nitrate sequihydrate, sancycline, lymecycline, mepicycline and minocycline;
5. phenols which are narcotic analgesics and narcotic antagonists selected from a group consisting of: morphine, apomorphine, hydromorphone hydrochloride, ketobemidone, morhine, morphine, hydrochloride, morphine N-oxide, nalorphine hydrochloride, naloxone hydroch-oride, pentazocine, levorphanol tartrate, metopon hydrochloride, buprenorphine hydrochloride, butorphanol, cyclazocine, profadol hydrochloride, levallorphan tartrate, alazocine, nalbufine hydrochloride, oxilorphan, nalmexone hydrochloride, naltrexone and apocodeine, cyclorphan, desomorphine, dihydromorphine, 3-hydroxy-N-methylmorphinane, hydroxypethidine, levophenacylmorphan, metazocine, norlevophanol, normorphine, oxymorphone, phenazocine, phenomorphan, pholcodine and cyprenorphine;
6. phenols which are anti-microbials including antibiotic/antibacterial, fungistatic, antimalarial and antiamebic agents selected from a group consisting of: 8-hydroxyquinoline, chlorquinaldol, 5-chloro-8-quinolinol, clamoxyquin, 8-hydroxy-7-iodo-5-quinolinesulfonic acid, iodochlorhydroxyquin, octoquin methylsulfate and oxyquinoline benzoate, rifamycin B, rifamycin SV, zanthocillin (X, $Y_1$ and $Y_2$) amoxicillin, celesticetin, chartreusin, novobiocin, resistomycin, rifamide, rifampin, albofungin, Formecin A, hygromycin, Acroteben[R], amidan, antranorin, azosulfamide, diathymosulfone, Flavoteben, 1-isonicotinoyl-2-salicylidene hydrazide, lasalocid, myxin, siccanin, tyrocidine A, tyrocidine B, tyrocidine C and actinorhordine;

$R^2$ is selected from the group consisting of straight or branched chain alkyl having from 1 to 20 carbon atoms; aryl having from 6 to 10 carbon atoms; cycloalkyl having from 3 to 8 carbon atoms; alkenyl having from 2 to 20 carbon atoms; cycloalkenyl having from 4 to 8 carbon atoms; alkynyl having from 2 to 20 carbon atoms; aralkyl, alkaryl, aralkenyl, aralkynyl, alkenylaryl, alkynylaryl, (lower acyloxy)alkyl and carboxyalkyl, wherein alkyl, aryl, alkenyl and alkynyl are as defined above; 3- to 8-membered, saturated or unsaturated monoheterocyclic or polyheterocyclic containing from 1 to 3 of any one or more of the heteroatoms N, S or O; and mono- or polysubstituted derivatives of the above, each of the substituents being selected from the group consisting of lower alkyl, lower alkoxy, lower acyl, lower acycloxy, halo, halo(lower alkyl), cyano, carbethoxy, lower alkylthio, amino, nitro, lower alkylamino, di(lower alkyl)amino, carboxyl, carbamyl, lower alkylcarbamyl, di(lower alkyl)carbamyl and

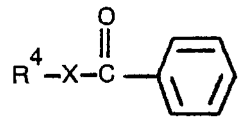

wherein $R^4$ is hydrogen or alkyl having from 1 to 10 carbons; $R^3$ is hydrogen, lower acyl, cyano, halo(lower alkyl), carbamyl, lower alkylcarbamyl, di(lower alkyl)carbamyl, $—CH_2ONO_2$, $—CH_2OCOR^2$, or any non-heterocyclic member of the group defined by $R^2$ above; and

*n* is at least one and equals the total number of phenolic hydroxyl functions comprising the $R^1$-phenol etherified via a $R^2COXCH(R^3)O—$ moiety; those having the structural formula (II):

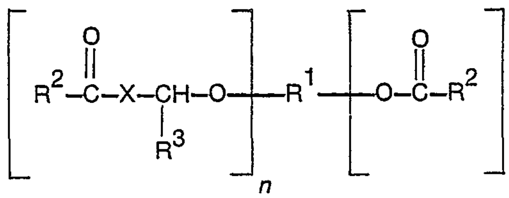 (II)

wherein X, $R^1$, $R^2$ and $R^3$ are as defined above, and *n* and *m* are each at least one and the sum of $n + m$ equals the total number of phenolic hydroxyl functions comprising the $R^1$-phenol either etherified via a $R^2COXCH(R^3)O—$ moiety or esterified via a $R^2COO—$ moiety; and the pharmaceutically acceptable acid addition salts, quaternary ammonium salts and N-oxides thereof.

By "monodehydroxylated" residue of a non-steroidal phenol as utilized herein, there are intended those monovalent radicals as would result upon removal of one of the phenolic hydroxyls from the nucleus of the parent benzenoid and leaving a free or unsatisfied valence bond in its stead, i.e., that valence bond which defines the bridge to the $R^2COXCH(R^3)O—$ moiety of the compounds of the structural formula (I). Similarly, the "polydehydroxylated" residue connotes those polyvalent radicals as would result upon removal of more than one nuclear hydroxyl from the parent aromatic compound. Thus, representative monovalent radicals include, for example:

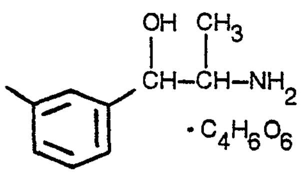

metaraminol bitartrate residue

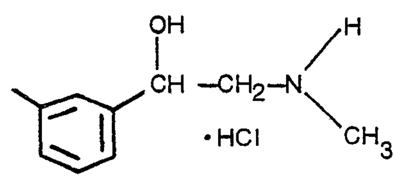

phenylephrine hydrochloride residue

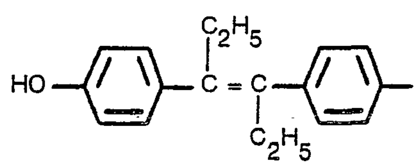

diethylstilbestrol residue

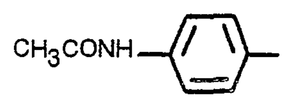

acetaminophen residue

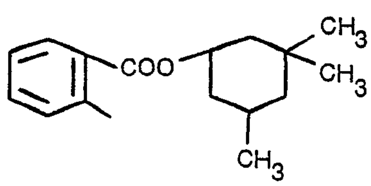

homosalate residue

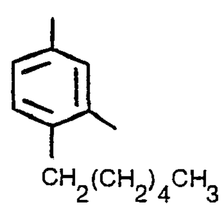

4-hexylresorcinol residue

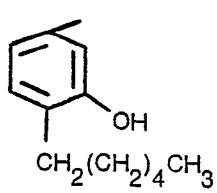

4-hexylresorcinol residue

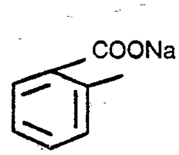

sodium salicylate residue

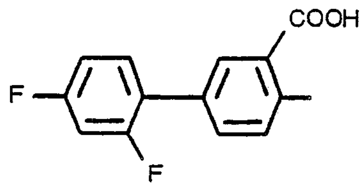

diflunisal residue

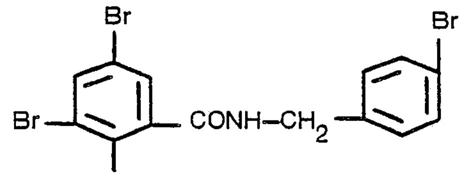

bensalan residue

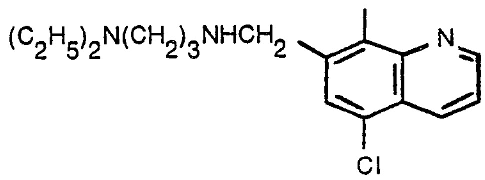

clamoxyquin residue

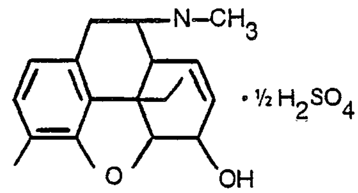

morphine sulfate residue

and representative polyvalent radicals correspondingly include:

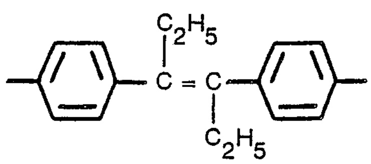

diethylstilbestrol residue

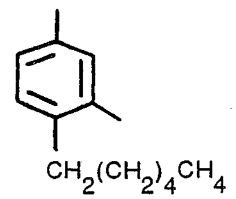

4-hexylresorcinol residue

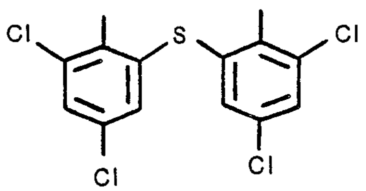

bithionol residue

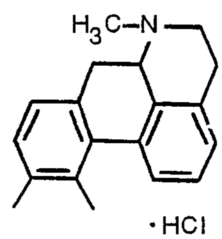

apomorphine hydrochloride residue

When $R^2$ comprises a heterocyclic function, representative such heterocycles include, without limitation, and with regard to the point of attachment on the ring, piperazinyl, 4-methylpiperazinyl, pyridinyl, pyrazinyl, pyrimidinyl, pyridazinyl, pyrazolyl, pyrrolyl, pyrrolidinyl, pyrrolinyl, pyrazolinyl, pyrazolidinyl, piperidyl, morpholinyl, quinuclidinyl, isoindolinyl, indolinyl, thienyl, benzothienyl, napthothienyl, thianthrenyl, furyl, pyranyl, chromenyl, xanthenyl, phenoxathiinyl, imidazolyl, pyridyl, indolizinyl, isoindolyl, 3H-indolyl, indolyl, indazolyl, purinyl, phthalazinyl, quinolyl, isoquinolyl, 4H-quinolizinyl, quinoxalinyl, naphthyridinyl, cinnolinyl, pteridinyl, carbazolyl, 4aH-carbazolyl, phenanthridinyl, acridinyl, perimidinyl, phenoxazinyl, furazanyl, isochromanyl, chromanyl, imidazolinyl, 1-methylazarinyl, 1-methylpyrrolyl, 1-methylimidazolyl, 1-methylpyrazolyl, 2-methylisoindolyl, 3H-indolyl, phtalazinyl, quinoxilinyl, quinazidinyl, phenazinyl, isothiazolyl, 10-methylphenothazinyl, isoxazolyl, furazanyl, the various saturated, unsaturated or partially saturated congeners of any of the above, and those attached to the carbonyl carbon via a lower alkylene bridge.

By "pharmaceutically acceptable salt", there are intended the conventional non-toxic acid addition or quaternary ammonium salts of the compounds of the formulae (I) and (II), formed, e.g., from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, fumaric, toluenesulfonic, and the like. Preferred salts of the invention are those derived from the same acids as are the generally preferred salts of the parent phenols, e.g. the hydrochlorides in the case of the phenylephrine derivatives.

The pharmaceutically acceptable salts of the present invention can be synthesized from the compounds embraced by formulae (I) and (II) by conventional chemical methods. Generally, the salts are prepared by reacting the free base with stoichiometric amounts of, or with an excess of, the desired salt-forming inorganic or organic acid in a suitable solvent or various combinations of solvents. For example, the free base can be dissolved in a mixed aqueous solution of the appropriate acid and the salt recovered by standard techniques, for example, by evaporation of the solution. Alternatively, the free base can be charged into an organic solvent such as lower alkanol, a symmetrical or asymmetrical ether containing 2 to 10 carbon atoms, an alkyl ester, or mixtures thereof, and the like, and then it is treated with the appropriate acid to form the corresponding salt. The salt is recovered by standard recovery techniques, for example, by filtration of the desired salt or spontaneous separation from the solution, or it can be precipitated by the addition of a solvent in which the salt is insoluble and recovered therefrom.

Examples of suitable inorganic and organic solvents for performing the various reactions include any inorganic or organic solvent that does not adversely affect the reactants or the resulting product, including halogenated solvents such as methylene chloride, chloroform, carbon tetrachloride, ethylene chloride, ether solvents such as diethyl ether, dimethyl ether, and other solvents such as tetrahydrofuran, dioxane, diglyme, n-hexane, cyclooctane, benzene, heptane, cyclohexane; mixtures thereof, and the like aliphatic, cycloaliphatic and aromatic hydrocarbon solvents, water, acidified aqueous solutions, mixed organic and inorganic solutions, ethyl acetate, propyl acetate, and the like.

The "quaternary ammonium salts" are likewise conventional to the pharmaceutical arts, and these too are prepared via typical methodology. Moreover, either the $R^1$ or the $R^2$ moiety, or both, of the subject prodrug molecules can be quaternized or otherwise comprise a salt function.

The compounds of the present invention are conveniently prepared via the following general synthesis:

When the desired compound of the invention contains *one* or more primary or secondary amine functions, eg. phenylephrine, metaraminol, etc., said function(s) is/are first suitably protected, e.g., with a t-butyloxycarbonyl protective group by reaction of the parent phenol with t-butylazidoformate, in the presence of base, e.g., triethylamine or N-methylmorpholine, in a polar aprotic solvent, e.g., dioxane, THF or dichloromethane, under atmospheric pressure and at a temperature for from −20°C to the boiling point of the solvent, preferably from 0°C to 20°C. Next, the N-t-butoxycarbonyl derivative which results is reacted, under $S_N2$ conditions, with at least *n* equivalents (but preferably an excess) of a compound of the formula:

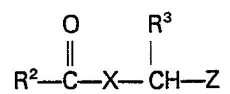

wherein X, $R^2$ and $R^3$ are as above defined and Z is a suitable leaving group, e.g., chloride, bromide, tosylate, etc., and preferably the iodide, in the presence of at least *n* equivalents (but preferably an excess) of a suitable base, e.g., potassium carbonate, in a ketone solvent, e.g. acetone, methylethylketone, cyclohexanone, 2-pentanone, 3-hexanone, or the like, to form the corresponding N-t-butoxycarbonyl derivative having *n* acyl-X-methyl ether moieties in place of the *n* nuclear hydroxy groups in the starting material, e.g., in the case of phenylephrine,

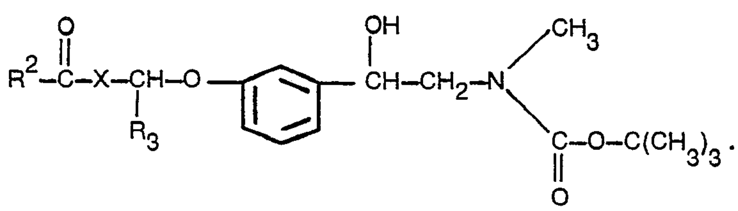

The t-BOC protective group is then removed by protonation with HA, e.g., with HCl in ethyl acetate, or trifluoroacetic acid in dichloromethane or tetrahydrofuran, or any "other" common reagent for removing the t-BOC protective group in amino acid chemistry, to result in the compound according to the invention, e.g., in the case of phenyleprine,

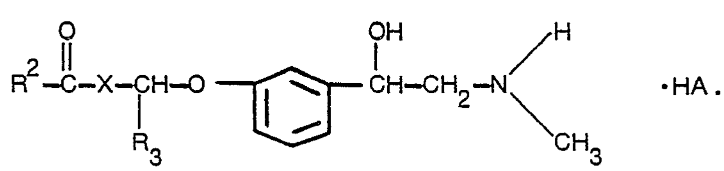

The reactant 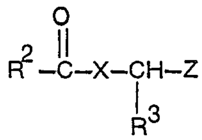, wherein X is either O, S or NH, is prepared thus:

(i) 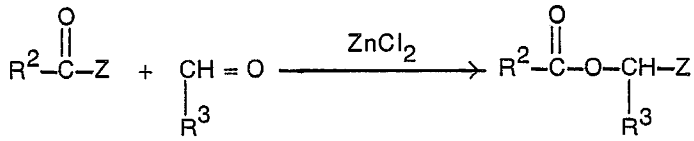


and, when X is Cl or Br, 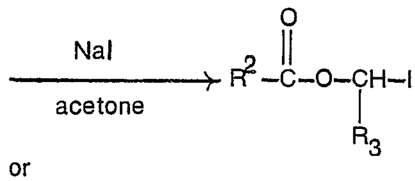


or

(ii) 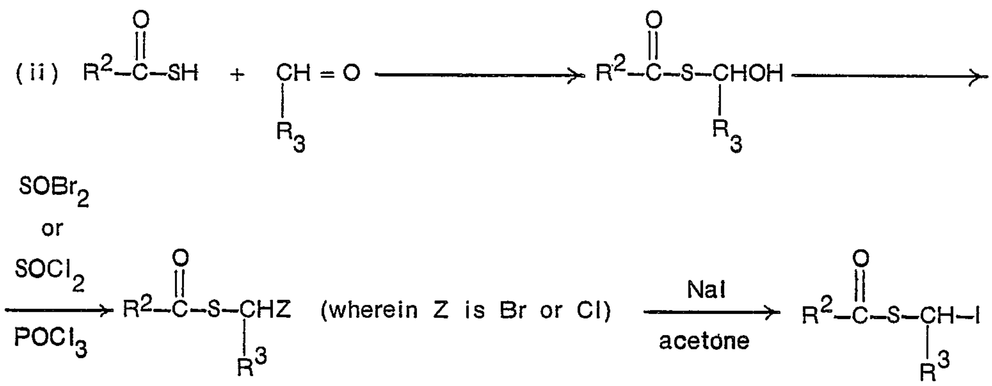


or

(iii) 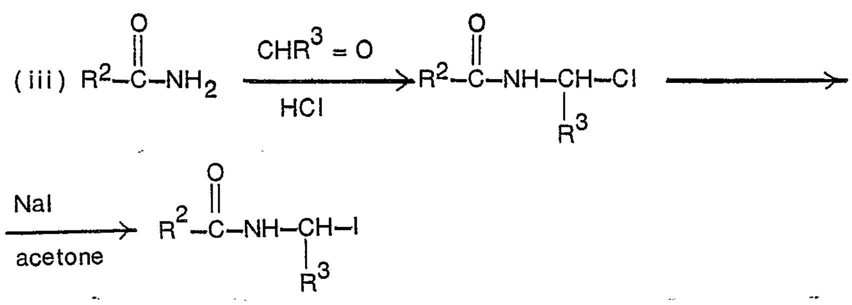


or

(iv) 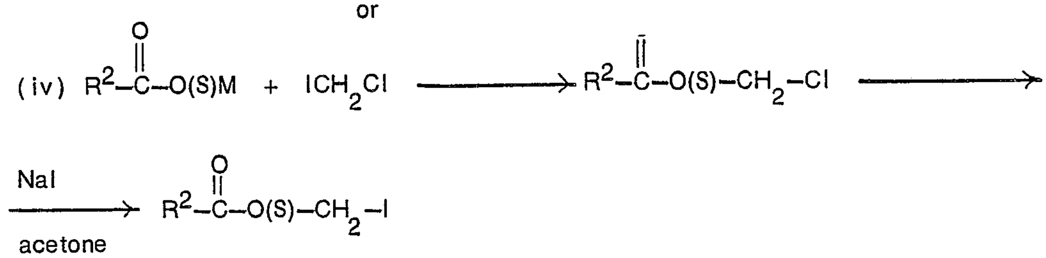


or

(v) 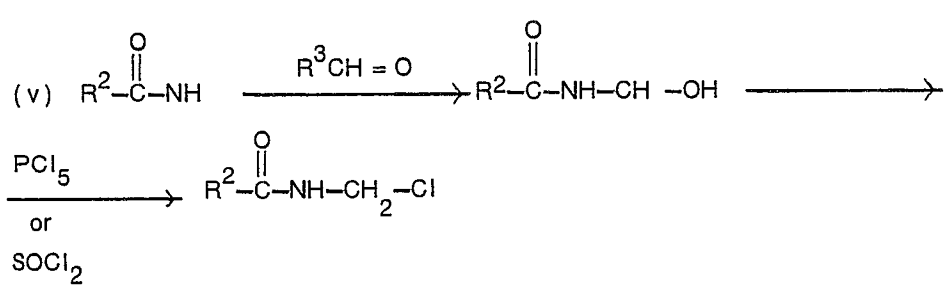

Other suitable N-protective groups, i.e., other than t-butoxycarbonyl or t-BOC protective group, include N-formate, carbobenzyloxy, $—Ch_2S—R$, $—COCF_3$, $—C(CH_3)=C=COCH_3$, any completely protected peptide, e.g., —CO—CH(R)NHCH=O, $=CH—C_6H_4$ (m—OH) [for, e.g., metaraminol], and the like. Similarly, when a benzyl hydroxyl function is present, although it is less reactive, it too may be protected, if desired, e.g., by lower acylation. The N-protected or O-protected compounds, thus, not only are useful intermediates, but are also useful final products, also demonstrating the utility of the parent drug species.

Other functional groups can, if desired, be protected in a similar manner. Thus, for example, when the desired compound of the invention contains one or more carboxylic acid functions, e.g., @-methyl-p-tyrosine, salicylic acid, salicylsalicylic acid, diflunisal, etc., the carboxyl function(s) is/are likewise protected prior to reaction with

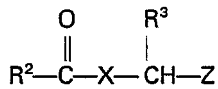

for example, by first reacting the parent phenol with benzyl alcohol and HCl. The free acid would then ultimately be obtained, e.g., by catalytic hydrogenation, after alkylation. Of course, any conventional protective group for the carboxy function other than benzyl could also be utilized. Alternatively, the —COOH group(s) can be left unprotected, in which case sufficient alkylating agent of the formula

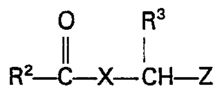

is used to esterify the —COOH groups while alkylating the —OH groups.

A representative synthetic scheme utilizing protecting groups is as follows:

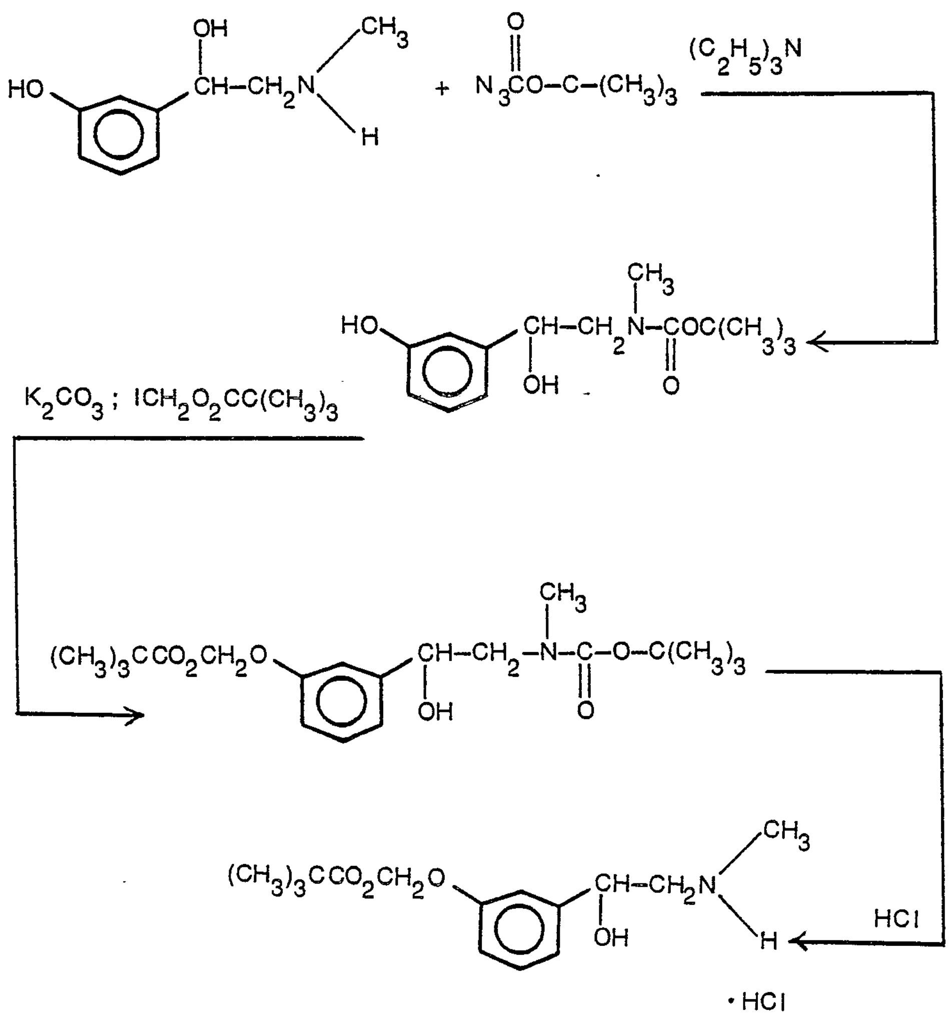

As a variation of the above synthetic scheme, when there are no amino or carboxy functions, the parent phenol of the formula

$$R^1\text{—}[OH]_n$$

wherein $R^1$ and *n* are defined as hereinabove, is simply reacted with at least *n* equivalents (but preferably an excess) of the alkylating agent of the formula

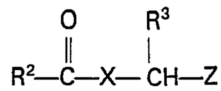

wherein X, $R^2$, $R^3$ and Z are defined as hereinabove, under the reaction conditions for the alkylation described *supra.* Alternatively, the parent phenol can first be converted to its alkali metal salt and the salt reacted with the desired alkylating agent. Exemplary of the reaction scheme when no protecting groups are needed are the reaction of potassium 4-acetamidophenolate with N-chloromethylbenzamide to form N-(4-acetamidophenoxymethyl)benzamide; the reaction of phenol with iodomethyl pivalate in the presence of potassium carbonate to afford α-pivaloylanisole; and the reaction of 4-hexylresorcinol with iodomethyl pivalate in the presence of potassium carbonate to form 4-hexyl-1,3-bis(pivaloyloxymethoxy)benzene.

The compounds of formula (I) wherein *n* is 2 can be partially hydrolyzed to afford the corresponding mono(acyl-X-methyl ether) derivatives, e.g., 4-hexyl-1,3-bis(pivaloyloxymethoxy)benzene can be hydrolyzed to

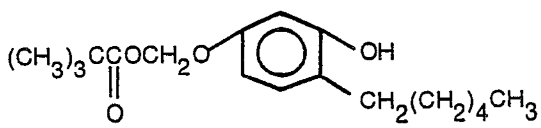

or to

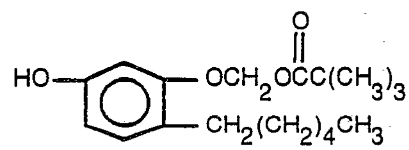

or to a mixture of both isomeric forms. The mono(acyl-X-methyl ether) derivatives may also be prepared by using much lesser amounts of the

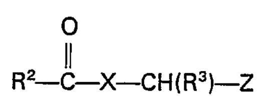

reactant in the alkylation process. In a similar manner, the compounds of formula (I) wherein *n* is greater than 2, e.g. 3 or 4, can be partially hydrolyzed to the less substituted mono(acyl-X-methyl ethers)/bis(acyl-X-methyl ethers)/tris(acyl-X-methyl ethers). And, as before, those less substituted derivatives likewise can be obtained by using less

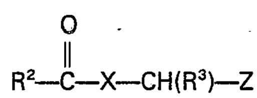

reactant in the alkylation.

The compounds of formula (I) which contain at least one free nuclear hydroxy group can be reacted with a suitable acylating agent, e.g., $R^2COCl$, to afford the corresponding compounds of formula (II). Alternatively, an appropriate starting material, for example an aldehyde starting material such as 3,4-dihydroxy benzaldehyde, can be reacted under the appropriate conditions, e.g., with

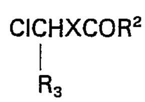

in the presence of $K_2CO_3$, to afford the part acylated, part acyl-X-methylated derivatives, e.g.

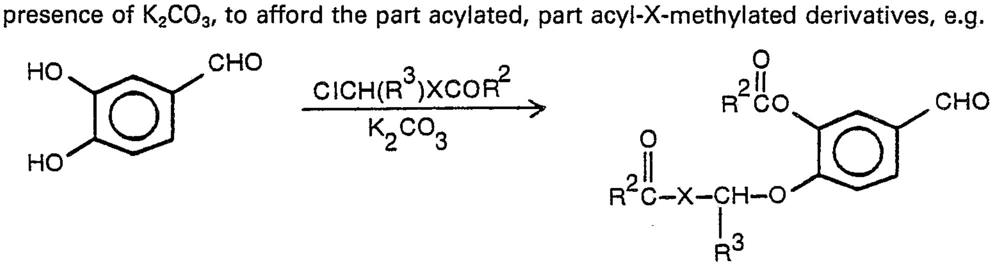

If desired, that product may then be converted to selected other compounds of this invention. The bis(acyl-X-methylated) derivatives of 3,4-dihydroxybenzaldehyde prepared under the usual alkylation conditions described hereinbefore, likewise may be converted to selected other compounds of this invention.

While all of the compounds according to the invention are characterized by good lipid solubility and high bioavailability, are quite stable to both air and light, and are more immune to chemical attack by those agents which are conventionally used in pharmaceutical preparations, the same are nonetheless facilely chemically and/or enzymatically metabolized/hydrolyzed at their sites of action, e.g., upon administration are cleaved into the known and proven parent drug molecule such as phenylephrine *per se,* as well as into various non-toxic products of metabolism/hydrolysis, according to the following general scheme:

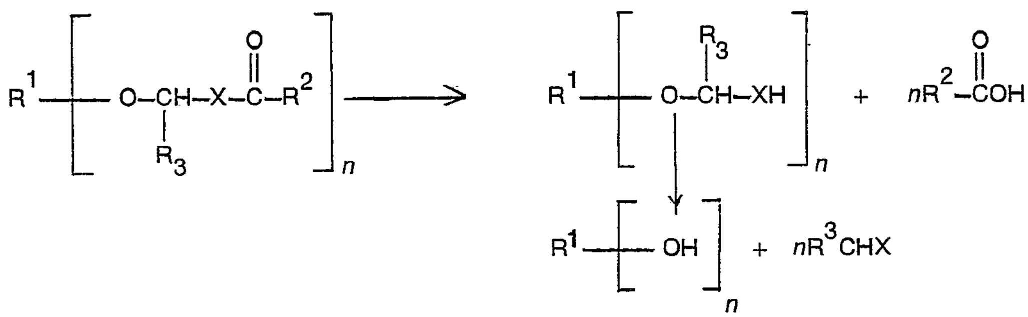

It will be appreciated that it is a critical feature of the present invention that the ether oxygen and the X function comprising the acyl-X-methyl ether moiety of the subject prodrug compounds be separated by but a single carbon atom or methylene bridge. Otherwise, e.g., if the "methylene" linkage were ethylene or higher alkylene, such compounds would not be subject to the aforesaid chemical and/or enzymatic metabolism/hydrolysis and would not be facilely cleaved *in vivo,* into the noted non-toxic products of metabolism/hydrolysis. Hence, such ethylene and higher alkylene congeners are inoperative and not intended herein; indeed, same could not properly be deemed or designated as true "prodrugs".

While all of the compounds encompassed within the aforesaid generic formulas meet applicants' criteria, nevertheless certain compounds remain preferred, namely, the pivaloyloxymethyl, hexanoyloxymethyl, heptanoyloxymethyl, octanoyloxymethyl, n-dodecanoyloxymethyl, n-tetradecanoyloxymethyl, n-hexadecanoyloxymethyl, acetyloxymethyl, pentanoyloxymethyl, benzoyloxymethyl, benzoyloxybenzyl, propionyloxymethyl, butyryloxymethyl, benzoylaminomethyl and pivaloylthiomethyl ether derivatives of the bio-affecting phenols of the formula $R^1—[OH]_n$ where *n* and $R^1$ are defined as before. Particularly preferred among these ethers are those of the nuclear monohydroxy sympathomimetic amines (metaraminol, phenylephrine, deterenol, etc.); of tetracyline-type antibiotics; of non-steroidal estrogens (e.g., diethyl-stilbestrol, dienestrol, etc.); of salicylic acid, acetaminophen, diflunisal and structurally related non-steroidal anti-inflammatory and non-narcotic analgesic agents; of morphine and structurally related narcotic analgesics and narcotic antagonists; and of phenol and structurally-related antimicrobials.

From the foregoing, it will be appreciated that the prodrug derivatives according to invention exhibit all of the biological and therapeutic activity of their "parent" drug species, for the treatment of whatever disease state or condition is known to be responsive to administration of the parent drug species (e.g., for glaucoma, bronchial asthma, nasal congestion, allergies, itching, inflammation, etc. in the case of the sympathomimetic amines) at the same time being characterized by enhanced bioavailability and physiological availability, enhanced resistance to deterioration by air and light and to chemical attack, and even the ability to elicit the same pharmacological response as the parent drug form, but at lower dosages.

The dose of the prodrug administered, whether orally, topically, intravenous solution, or the like, and whether a single dose or a daily dose, will, of course, vary with the needs of the individual. Moreover, the dosage administered is not subject to definite bounds, but will usually be an effective amount, or the equivalent on a molar basis of the pharmacologically active form produced upon the metabolic release of the active, parent drug species to achieve its desired and physiological effect, for example, depending on the nature of the parent drug, a sympathometically effective amount, an anti-inflammatory effective amount, an antibiotic effective amount, an estrogenically effective amount, etc. of the selected prodrug. See *Physicians' Desk Reference, 31* (1977). Moreover, for any of the broad spectrum of dosage forms into which the subject prodrugs can be formulated, i.e., any of the dosage forms into which the parent phenols can be formulated, see *Remington's Pharmaceutical Sciences,* 14th Edition (1970). Likewise, for those compounds of the invention derived from parent phenols which are not used as pharmaceuticals but are otherwise bio-affecting, e.g., the veterinary coccidiostats, the disinfectants such as phenol or any of the other bio-affecting non-pharmaceutical phenols, same can be formulated into any of the well known compositions into which the parent phenols are formulated and can be used for the same purposes.

Without further elaboration, it is believed that one of ordinary skill in the art can, using the preceding description, utilize the present invention to its fullest extent. Therefore, the following examples are to be construed as merely illustrative and not limitative of the remainder of the specification and claims in any way whatsoever.

Example 1

N-(4-Acetamidophenoxymethyl)benzamide

Potassium 4-acetamidophenolate is prepared by reacting 1.50 grams (0.01 mole) of 4-acetamidophenol with 0.7 gram (0.0105 mole) of 85% potassium hydroxide in 15 ml of methanol. The methanol is evaporated and the residue is suspended in 50 ml of tetrahydrofuran containing 2 ml of dimethylformamide. That suspension is combined with 1.85 grams (0.011 mole) of N-chloromethylbenzamide and allowed to react overnight at room temperature. The reaction mixture is then filtered and the filtrate is concentrated. The resulting residue is suspended in acetone and that suspension is filtered. The filtrate is adsorbed on silica gel and chromatographed on silica gel (56 grams) using methanol in ether (1000 ml of 1% and 1000 ml of 10%) as the eluent to give a fraction containing the desired compound with 4-acetamidophenol as a contaminant. The fraction is concentrated and the residue (1.1 grams) that results is triturated with 100 ml of ethyl ether and filtered to give 0.35 gram (melting point 157—164°C, 13% yield) of the desired N-(4-acetamidophenoxymethyl)benzamide: analytical sample, melting point 174—175°C from acetone; IR (KBr) 3300 $cm^{-1}$ (sharp, s) (N—H) 3300—3000 $cm^{-1}$ (broad, m) (N—H) and 1665 and 1645 $cm^{-1}$ (s) (C=O); $^1H$ NMR (DMSO-$d_6$) δ 9.83—9.17 (m, 1, N—<u>H</u>), 9.0—8.6 (m, l, N—<u>H</u>), 7.9—7.6 (m, 2, aromatic-*H*), 7.6—7.25 (m, 3, aromatic-*H*), 6.77 (AB quartet, 4, 3000 $cm^{-1}$ $J$=8Hz, $\Delta_{AB}$=19Hz, aromatic-*H*), 5.15 (d, 2, $J$=6Hz, NHC<u>H</u>$_2$O) and 1.73 (s, 3, C<u>H</u>$_3$CO).

*Anal.* Calcd for $C_{16}H_{16}N_2O_3$: C, 67.59; H, 5.67; N, 9.86. Found: C, 67.40; H, 5.70; N, 9.80.

The product has the formula

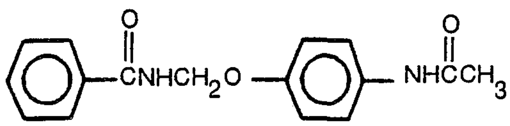

Example 2

(a) 3-Hydroxy-α-[(N-methyl-N-*tert*-butoxycarbonyl)aminomethyl]benzyl alcohol

To a suspension of phenylephrine (10 grams, 60 mmol) in 100 ml of water, 200 ml of p-dioxane and 100 ml of triethylamine is added *t*-butylazidoformate (10 grams, 65 mmol). After stirring for 18 hour at room temperature under $N_2$, the solution is concentrated *in vacuo.* The residue is taken up in ether and 1 *N* HCl. The acid layer is discarded and the ether layer is washed again with 1 *N* HCl and saturated sodium bicarbonate solution. After drying over anhydrous magnesium sulfate, the solvent is removed *in vacuo* to yield 13.5 g (84%) of a clear glass which is crystallized from mixtures of benzene and hexane to afford white cubes of the desired product, i.e., 3-hydroxy-α-[(N-methyl-N-*tert*-butoxycarbonyl)aminomethyl]benzyl alcohol, melting at 88—90°C. That product, which can also be called N-*t*-butyloxycarbonylphenylephrine, has the structural formula

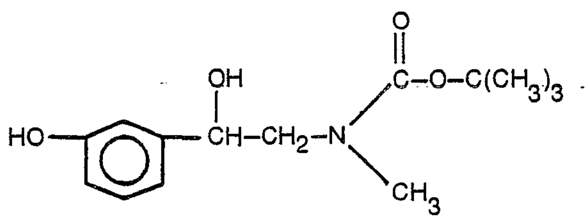

and is further characterized as follows:

NMR ($CDCl_3$) δ 1.43 (s, 9, O—C (C<u>H</u>$_3$)$_3$), 2.81 (s, 3, N—C<u>H</u>$_3$), 3.30—3.65 (m, 2, C—C<u>H</u>$_2$—N), 4.78 (b, l, —C*H*—O), 6.65—7.40 (m, 5, Ar—H and Ar—OH); IR (KBr) 3340, 3200, 1600 $cm^{-1}$.

*Anal.* Calc: C, 62.90; H, 7.92; N, 5.24. Found: C, 62.96; H, 8.25; N, 5.18.

(b) α-[(N-Methyl-N-*tert*-butoxycarbonyl)aminomethyl-3-(pivaloyloxymethoxy)benzyl alcohol

A suspension of 3-hydroxy-α-[(N-methyl-N-*tert*-butoxy-carbonyl)aminomethyl]benzyl alcohol (2.5 grams, 9.4 mmol), iodomethyl pivalate (4 grams, 16.5 mmol) and potassium carbonate (2 grams, 14.5 mmol) is stirred for 18 hours at room temperature, under nitrogen. The solvent is removed *in vacuo* and the residue is taken up in ether. The inorganic solids are removed by filtration and the ether is evaporated *in vacuo* to give a clear oil, which is purified by chromatography over silica gel using 20% ethyl acetate in benzene as eluant to yield, as a clear syrup, 2.9 grams of α-[(N-methyl-N-*tert*-butoxycarbonyl)aminomethyl]-3-(pivaloyloxymethoxy)benzyl alcohol. The product has the structural formula

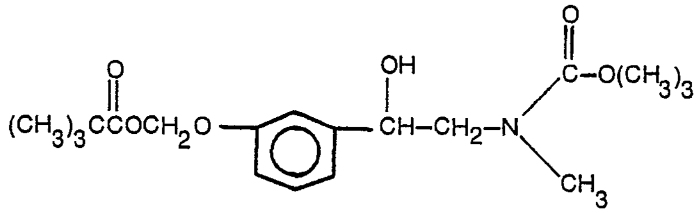

and is characterized as follows: NMR ($CDCl_3$) δ 1.18 (s, 9, $\overset{O}{\overset{\|}{C}}C(C\underline{H}_3)_3$), 1.44 (s, 9, —O—$C(C\underline{H}_3)_3$), 2.81 (s, 3, N—$C\underline{H}_3$), 3.44 (d, 2, C—$C\underline{H}_2$—N), 4.90 (t, l, O—C$\underline{H}$—), 5.75 (s, 2, O—$C\underline{H}_2$—O), 6.80—7.45 (m, 4, Ar—H); IR (film) 3430, 2990, 1750, 1675 $cm^{-1}$ (C=O).

*Anal.* Calc. for $C_{20}H_{31}NO_6$: C, 62.97; H, 8.19; N, 3.67. Found: C, 62.89; H, 8.60; N, 3.58.

(c) α-(N-Methyl)aminomethyl-3-(pivaloyloxymethoxy)benzyl alcohol hydrochloride

Anhydrous hydrogen chloride is bubbled through a solution of α-[(N-methyl-N-*tert*-butoxycarbonyl)-aminomethyl]-3-(pivaloyloxymethoxy)benzyl alcohol (0.38 grams, 1.1 mmol) dissolved in 10 ml of ethyl acetate for 5 minutes at 0°C. The solvent and excess acid are removed *in vacuo* to yield, as a glass, α-(N-methyl)aminomethyl-3-(pivaloyloxymethoxy)-benzyl alcohol hydrochloride. The product, which is homogeneous as shown by thin layer chromatography (chloroform-methanol, 80:20), has the formula

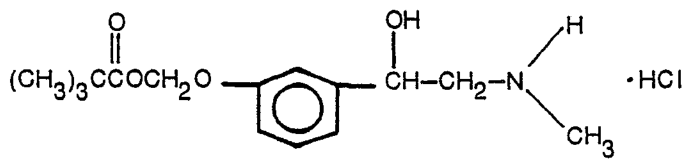

and is further characterized as follows: NMR ($CDCl_3$) δ 1.16 (s, 9, $\overset{O}{\overset{\|}{C}}C(C\underline{H}_3)_3$, 2.77 (b, 3, N—$C\underline{H}_3$), 3.12 (b, 2, C—$C\underline{H}_2$—N), 5.30 (b, l, O—C$\underline{H}$—C—), 5.68 (s, 2, O—$C\underline{H}_2$—O), 6.80—7.20 (m, 4, Ar*H*); IR (film) 3300 (broad), 1740, 1600, 1455, 1470 $cm^{-1}$.

The following compounds are also prepared utilizing the processes generally or specifically described herein, for example, the methods of Example 1, or their obvious chemical equivalents:

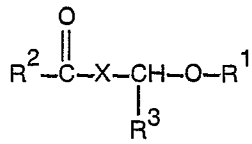

| Example Number | $R^1$ = the mono dehydroxylated residue of | $R^2$ | $R^3$ | X |
|---|---|---|---|---|
| 3 | thymol | $(CH_3)_3C-$ | $H_3C-$ | S |
| 4 | dibromsalan | 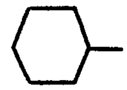 | H | S |
| 5 | chlorophene | $CH_3CH_2-$ | 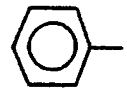 | O |
| 6 | phentolamine | $H_{23}C_{11}$ | H | O |
| 7 | eugenol | $(CH_3)_3C-$ | H | O |
| 8 | saligenin | 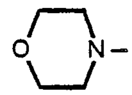 | H | O |
| 9 | oxyphenbutazone | 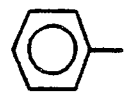 | $H_3C-$ | S |
| 10 | morphine | $(CH_3)_3C-$ | H | O |
| 11 | homosalate | 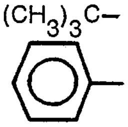 | $H_3C-$ | S |
| 12 | sodium salicylate | 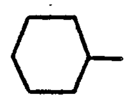 | $H_3C-$ | O |
| 13 | diflunisal | 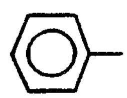 | H | O |
| 14 | bensalan | $(CH_3)_3C-$ | H | O |
| 15 | benzestrol | $(CH_3)_3C-$ | H | O |
| 16 | biphenamine | $(CH_3)_3C-$ | H | O |
| 17 | 5-chloro-8-quinolinol | 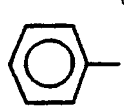 $-CH = CH_2-$ | H | O |
| 18 | diiodohydroxyquin | $(CH_3)_3C-$ | H | O |
| 19 | iodochlorhydroxyquin | 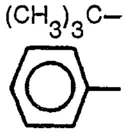 | H | S |
| 20 | methyl salicylate | $(CH_3)_3C-$ | 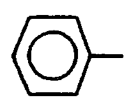 | S |
| 21 | nalorphine | $(CH_3)_3C-$ | H | S |
| 22 | naloxone | 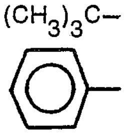 | 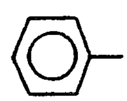 | O |
| 23 | oxyphenbutazone | $(CH_3)_3C-$ | H | O |

| Example Number | $R^1$ = the mono dehydroxylated residue of | $R^2$ | $R^3$ | X |
|---|---|---|---|---|
| 24 | pentazocine | $(CH_3)_3C-$ | H | NH |
| 25 | phenazocine | 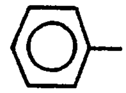 | H | O |
| 26 | salicylamide | 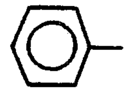 | H | O |
| 27 | diflunisal | $(CH_3)_3C-$ | H | S |

The following compounds of the invention are also prepared utilizing the processes generally or specifically described herein, for example, the methods of Example 2 or their obvious chemical equivalents. It will be apparent to those skilled in the art that the quantities of reactants will vary with the number of protecting groups needed. For example, when the $R_1$ residue contains a free $NH_2$ group, or when it contains two secondary amino groups, at least two moles of *t*-butylazidoformate are used in step (a) of the process of Example 2 per mole of phenolic starting material, and sufficient acid is utilized is step (c) so as to remove both protecting groups.

| Example Number | $R^1$ = the mono-dehydroxylated residue of | $R^2$ | $R^3$ | X |
|---|---|---|---|---|
| 28 | serotonin | $(CH_3)_3C-$ | H | O |
| 29 | metaraminol | $(CH_3)_3C-$ | H | O |
| 30 | phenylephrine | 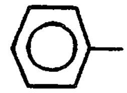 | H | O |
| 31 | α-methyl-p-tyrosine | 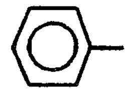 | H | O |
| 32 | phenylaminosalicylate | $(CH_3)_3C-$ | H | O |
| 33 | thyroxine | $(CH_3)_3C-$ | H | O |
| 34 | tyramine | 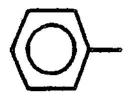 | $H_3C-$ | O |
| 35 | phenylephrine | $H_3C-$ | 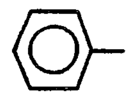 | NH |
| 36 | Vitamin $K_5$ | $(CH_3)_3C-$ | H | O |
| 37 | hydroxyamphetamine | $(CH_3)_3C-$ | H | O |
| 38 | albuterenol | $(CH_3)_3C-$ | H | O |
| 39 | deterenol | $(CH_3)_3C-$ | H | NH |
| 40 | quinterenol | $(CH_3)_3C-$ | H | S |
| 41 | isoxsuprine | 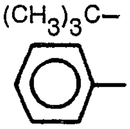 | H | O |
| 42 | nylidrin | 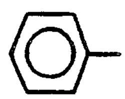 | H | O |
| 43 | L-α-aspartyl-L-tyrosine methyl ester | $(CH_3)_3C-$ | H | O |
| 44 | clamoxyquin | $(CH_3)_3C-$ | H | O |
| 45 | metaraminol | $(CH_3)_3C-$ | H | S |
| 46 | phenylephrine | 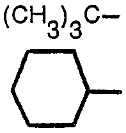 | H | NH |
| 47 | metaraminol | 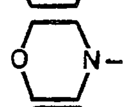 | $H_3C-$ | S |
| 48 | albuterenol | 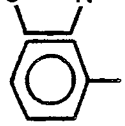 | H | NH |
| 49 | deterenol | 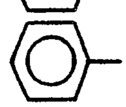 | H | O |

| Example Number | $R^1$ = the mono-dehydroxylated residue of | $R^2$ | $R^3$ | X |
|---|---|---|---|---|
| 50 | oxymetazoline | $(CH_3)_3C-$ | H | O |
| 51 | pirbuterol | 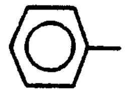 | 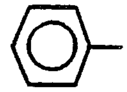 | O |
| 52 | isoxuprine | $(CH_3)_3C-$ | H | O |
| 53 | nylidrin | $(CH_3)_3C-$ | H | O |
| 54 | bamethan | 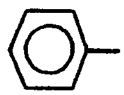 | $H_3C-$ | O |
| 55 | phenylephrine | $H_{25}C_{12}-$ | $H_3C-$ | O |
| 56 | phenylephrine | 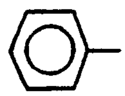 $-CH=CH_2-$ | H | S |
| 57 | tetracycline* | $(CH_3)_3C-$ | H | O |
| 58 | methacycline* | 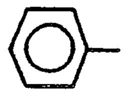 | H | O |
| 59 | chlortetracycline* | $(CH_3)_3C-$ | H | O |
| 60 | doxycycline* | $(CH_3)_3C-$ | H | O |

*Although each of these compounds technically contains only one phenolic hydroxy group, each of them also contains two other hydroxy groups attached to carbon atoms which are involved in double bond formation and which thus also have phenolic-type reactivity. Sufficient acylating agent is employed in step (b) so as to acylate all three of these hydroxy groups in each compound. The ultimate products are depicted below:

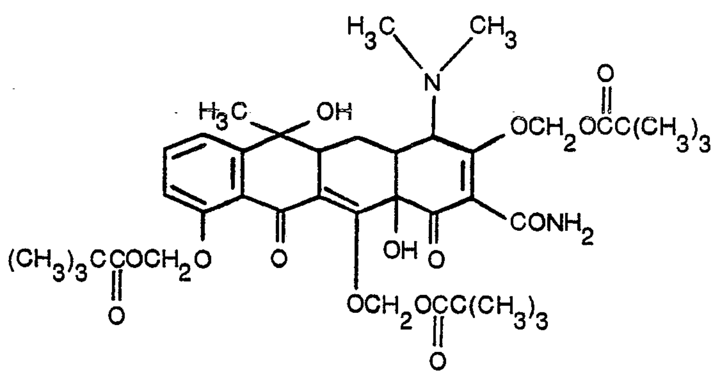

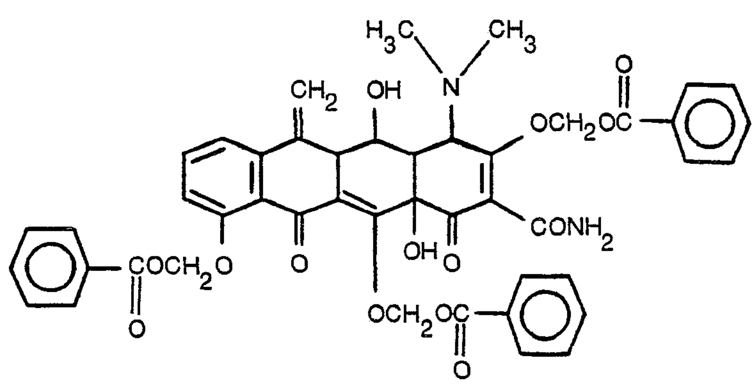

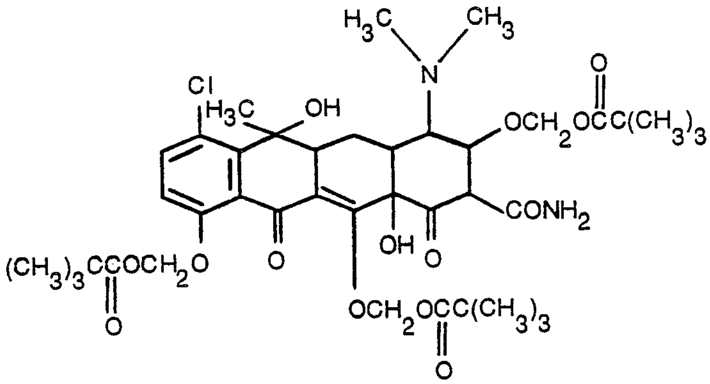

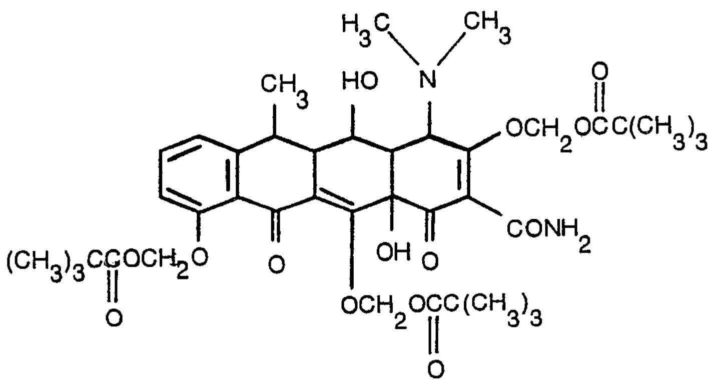

The following compounds are likewise pepared utilizing the processes generally or specifically described herein, for example, the method of Example 5, or its obvious chemical equivalent. The amount of acylating agent employed will of course vary with the number of phenolic hydroxy groups in the parent bioaffecting phenol.

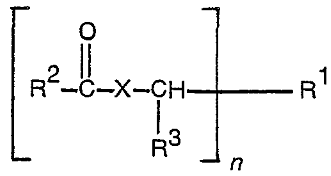

| Example Number | $R^1$ = the poly-dehydroxylated residue of | $R^2$ | $R^3$ | X | n |
|---|---|---|---|---|---|
| 61 | 4-hexylresorcinol | $(CH_3)_3C-$ | H | O | 2 |
| 62 | dichlorophene | $(CH_3)_3C-$ | $H_3C-$ | O | 2 |
| 63 | hexachlorophene | 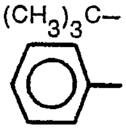 | H | O | 2 |
| 64 | hydroquinone | $(CH_3)_3C-$ | H | O | 2 |
| 65 | apomorphine | 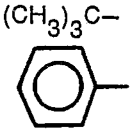 | H | O | 2 |
| 66 | diethylstilbestrol | $(CH_3)_3C-$ | H | O | 2 |
| 67 | bithionol | $(CH_3)_3C-$ | H | O | 2 |
| 68 | dienestrol | 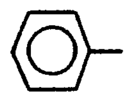 | 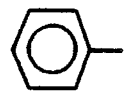 | O | 2 |
| 69 | hexestrol | $H_3C-$ | $H_3C-$ | NH | 2 |
| 70 | resorcinol | 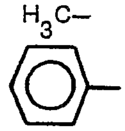 | H | S | 2 |
| 71 | dithranol | 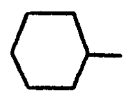 | $F_3C-$ | O | 3 |
| 72 | ellagic acid | 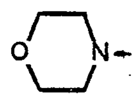 | 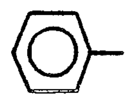 | O | 4 |
| 73 | eriodictyol | $H_{25}C_{12}-$ | H | O | 4 |
| 74 | fenticlor | $CH_3CH_2-$ | H | O | 2 |
| 75 | Formecin A | 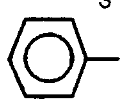 $-CH=CH_2-$ | H | O | 3 |
| 76 | frangulin | $(CH_3)_3C-$ | H | O | 2 |
| 77 | gallacetophenone | $(CH_3)_3C-$ | H | O | 3 |
| 78 | resistomycin | $(CH_3)_3C-$ | H | O | 4 |
| 79 | rutin | 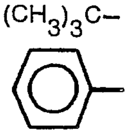 | H | O | 4 |
| 80 | salicil | $H_3C-$ | H | O | 2 |

| Example Number | $R^1$ = the poly-dehydroxylated residue of | $R^2$ | $R^3$ | X | n |
|---|---|---|---|---|---|
| 81 | xanthocillin X | $(CH_3)_3C-$ | H | O | 2 |
| 82 | xanthocillin $Y_1$ | 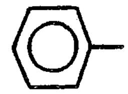 | H | S | 3 |
| 83 | xanthocillin $Y_2$ | $H_3C-$ | $H_3C-$ | O | 4 |

Example 84

3-Pivaloyloxy-4-pivaloyloxymethoxybenzaldehyde

3,4-Dihydroxybenzaldehyde (27.6 grams, 0.2 mol) is dissolved in 100 ml of dimethylformamide with 45 ml of triethylamine and 40 ml of pivaloyloxymethyl chloride. The mixture is stirred at room temperature for five days. After one day, 20 ml of pivaloyloxymethyl chloride and 20 ml of triethylamine are added to the mixture. The mixture is then poured into 600 ml of water. The water is extracted with 600 ml of methylene chloride and twice with 500 ml of ether. The organic layers are concentrated and that residue is chromatographed on silicAr CC—7 using petroleum ether (b.p. 30—60°C)/acetone (1:1) as eluant to give the desired 3-pivaloyloxy-4-pivaloyloxymethoxybenzaldehyde melting at 58—60°C.

**Claims for the Contracting States: BE CH DE FR GB IT LI LU NL SE**

1. A compound selected from the group consisting of those having the structural formula (I):

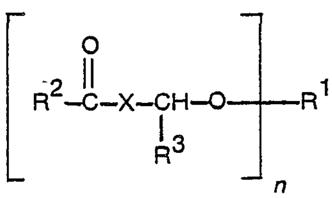

(I)

wherein

X is O, S or $NR^5$ wherein $R^5$ is hydrogen or lower alkyl;

$R^1$ is the nuclear mono- or polydehydroxylated residue of

1. a phenol selected from a group consisting of: hydroxyamphetamine, hydroxyamphetamine-hydrobromide, metaraminol, metaraminol bitartrate, phenylephrine hydrochloride, oxymetazoline hydrochloride, albuterenol (albuterol), carbuterol hydrochloride, deterenol hydrochloride, quinterenol sulfate, soterenol, sulfonterol hydrochloride, isoxuprine hydrochloride (isoxsuprine), nylidrin hydrochloride, bamethan, bamethan sulfate, mesuprine hydrochloride and ritodrine hydrochloride, benserazide, dimethophrine, *dl*-etilefrin, hordenine, p-hydroxyephedrine, ifenprodel, leptodactyline chloride, leptodactyline picrate, norfenefrine, octopamine, phentolamine, pholedrine, synephrine, tyramine and ethamivan;

2. a phenol which is a salicylic acid derivative or a structurally related compound selected from a group consisting of: acetaminophen, eugenol, p-aminosalicylic acid, 4-benzamidosalicylic acid, calcium benzoylpas, biphenamine, buclosamide, homosalate, hydroxyprocaine, methyl salicylate, oxybenzone, dioxybenzone, phenyl aminosalicylate, salicyl alcohol (saligenin), salicylamide, salicylic acid, salicylsalicylic acid (salsalate), sodium salicylate, difunisal, dibromsalan, fursalan, metabromsalan, bensalan, salethamide, and salcolex, p-aminosalicylic acid hydrazide, ammonium salicylate, 5-bromo-4'-chlorosalicylanilide, 5-bromosalicylhydroxamic acid, p-butylaminosalicylic acid 2-diethylaminoether ester, 3'-carboxy-4'-hydroxycinchophen, choline salicylate, 3,5--dibromosalicylaldehyde, fluorosalan, flopropione, gallacetophenone, genisic acid, 4-hydroxyisophthalic acid, hydroxytetracaine, N-isopropylsalicylamide, niclosamide, osalmid, oxyclozanide, oxyphenbutazine, Pascaine® (hydroxyprocaine p-aminosalicylate), phenetsal, phenyl salicylate, resorantel, salacetamide, salicyl, salicylanilide, salazosulfadimidine, salazosulfamide, salicylazosulfapyridine, 4-salicyloxymorphine, 4-sulfanilamidosalicylic acid, o-thymotic acid, 3',4',5'-trichlorosalicylanilide, xipamide, amidan, bromosaligenin and 3,5-diiodosalicylic acid;

3. a non-steroidal estrogen phenol selected from a group consisting of: benzestrol, dinestrol, diethyl stilbestrol, hexestrol, mestilbol and promethestrol;

4. a phenol of tetracycline-type antibiotics selected from a group consisting of: tetracycline, tetracycline-hydrochloride, chlortetracycline, demeclocycline, doxycycline, oxytetracycline, apicycline, clomocycline,

guamecycline, methacycline, rolitetracycline, rolitetracycline nitrate sesquihydrate, sancycline, lymecycline, mepicycline and minocycline;

5. phenols which are narcotic analgesics and narcotic antagonists selected from a group consisting of: morphine, apomorphine, hydromorphone hydrochloride, ketobemidone, morhine, morphine hydrochloride, morphine N-oxide, nalorphine hydrochloride, naloxone hydrochloride, pentazocine, levorphanol tartrate, metopon hydrochloride, butrenorphine hydrochloride, butorphanol, cyclazocine, profadol hydrochloride, levallorphan tartrate, alazocine, nalbufine hydrochloride, oxilorphan, nalmexone hydrochloride, naltrexone and apocodeine, cyclorphan, desomorphine, dihydromorphine, 3-hydroxy-N-methylmorphinane, hydroxypethidine, levophenacylmorphan, metazocine, norlevophanol, normorphine, oxymorphone, phenazocine, phenomorphan, pholcodine and cyprenorphine;

6. phenols which are anti-microbials including antibiotic/antibacterial, fungistatic, antimalarial and antiamebic agents selected from a group consisting of: 8-hydroxyquinoline, chlorquinaldol, 5-chloro-8-quinolinol, clamoxyquin, 8-hydroxy-7-iodo-5-quinolinesulfonic acid, iodochlorhydroxyquin, octoquin methylsulfate and oxyquinoline benzoate, rifamycin B, rifamycin SV, xanthocillin (X, $Y_1$ and $Y_2$) amoxicillin, celesticetin, chartreusin, novobiocin, resistomycin, rifamide, rifampin, albofungin, Formecin A, hygromycin, Acroteben®, amidan, antranorin, azosulfamide, diathymosulfone, Flavoteben, 1-isonicotinoyl-2-salicylidene hydrazide, lasalocid, myxin, siccanin, tyrocidine A, tyrocidine B, tyrocidine C and actinorhordine;

$R^2$ is selected from the group consisting of straight or branched chain alkyl having from 1 to 20 carbon atoms; aryl having from 6 to 10 carbon atoms; cycloalkyl having from 3 to 8 carbon atoms; alkenyl having from 2 to 20 carbon atoms; cycloalkenyl having from 4 to 8 carbon atoms; alkynyl having from 2 to 20 carbon atoms; aralkyl, alkaryl, aralkenyl, aralkynyl, alkenylaryl, alkynylaryl, (lower acyloxy)alkyl and carboxyalkyl, wherein alkyl, aryl, alkenyl and alkynyl are as defined above; 3- to 8-membered, saturated or unsaturated monoheterocyclic or polyheterocyclic containing from 1 to 3 of any one or more of the heteroatoms N, S or O; and mono- or polysubstituted derivatives of the above, each of the substituents being selected from the group consisting of lower alkyl, lower alkoxy, lower acyl, lower acyloxy, halo, halo(lower alkyl), cyano, carbethoxy, lower alkylthio, amino, nitro, lower alkylamino, di(lower alkyl)amino, carboxyl, carbamyl, lower alkylcarbamyl, di(lower alkyl)carbamyl and

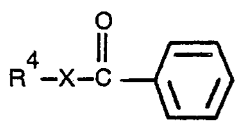

wherein $R^4$ is hydrogen or alkyl having from 1 to 10 carbons; $R^3$ is hydrogen, lower acyl, cyano, halo(lower alkyl), carbamyl, lower alkylcarbamyl, di(lower alkyl)carbamyl, $-CH_2ONO_2$, $-CH_2OCOR^2$, or any non-heterocyclic member of the group defined by $R^2$ above; and

*n* is at least one and equals the total number of phenolic hydroxyl functions comprising the $R^1$-phenol etherified via a $R^2COXCH(R^3)O-$ moiety; those having the structural formula (II):

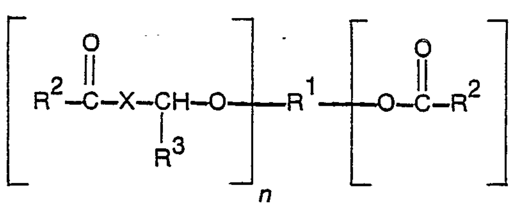

(II)

wherein X, $R^1$, $R^2$ and $R^3$ are as defined above, and *n* and *m* are each at least one and the sum of $n + m$ equals the total number of phenolic hydroxyl functions comprising the $R^1$-phenol either etherified via a $R^2COXCH(R^2)O-$ moiety or esterified via a $R^2COO-$ moiety; and the pharmaceutically acceptable acid addition salts, quaternary ammonium salts and N-oxides thereof.

2. A compound as defined by claim 1, wherein the *n* is 1, 2, 3, or 4.

3. A compound as defined by Claim 1, having the structural formula (II), wherein *n* is 1 and *m* is l.

4. A compound as defined by Claim 1, wherein $R^2$ is straight or branched chain alkyl having from 1 to 20 carbon atoms, aryl having from 6 to 10 carbon atoms, cycloalkyl having from 3 to 8 carbon atoms, alkenyl having from 2 to 20 carbon atoms, cycloalkenyl having from 4 to 8 carbon atoms or alkynyl having from 2 to 20 carbon atoms.

5. A compound as defined by Claim 1, wherein $R^3$ is hydrogen, alkyl, aryl or halo(lower alkyl).

6. A compound as defined by Claim 1, wherein $R^1$ is the monodehydroxylated residue of a nuclear monohydroxy sympathomimetic amine selected from the group consisting of hydroxyamphetamine, metarminol and phenylephrine, oxymetazoline, albuterenol, carbuterol, deterenol, quinterenol, soterenol, sulfonterol, isoxuprine, nylidrin, bamethan, mesuprine and ritodrine.

7. A compound as defined by Claim 1, wherein $R^1$ is
(a) the mono- or polydehydroxylated residue of a nuclear mono- or trihydroxy sympathomimetic amine selected from the group consisting of benserazide, dimethophrine, *dl*-etilefrin, hordenine, p-hydroxyephedrine, ifenprodel, leptodactyline, norfenefrine, octapamine, phentolamine, pholedrine, synephrine, tyramine and ethamivan;
(b) the mono- or polydehydroxylated residue of a non-steroidal bio-affecting phenol selected from the group consisting of acetaminophen, eugenol, p-aminosalicylic acid, 4-benzamidosalicylic acid, calcium benzoylpas, biphenamine, buclosamide, homosalate, hydroxyprocaine, methyl salicylate, oxybenzone, dioxybenzone, phenyl aminosalicylate, salicyl alcohol, salicylamide, salicylic acid, salicylsalicylic acid, sodium salicylate, diflunisal, octbenzone, salethamide, salcolex, p-aminosalicylic acid hydrazide, ammonium salicylate, 5-bromo-4'-chlorosalicylanilide, 5-bromosalicylhydroxamic acid, p-butylaminosalicylic acid 2-diethylaminoethyl ester, 3'-carboxy-4'-hydroxycinchopen, choline salicylate, 3,5-dibromosalicylaldehyde, flopropione, gallacetophenone, gentisic acid, 4-hydroxyisophthalic acid, hydroxytetracaine, N-isopropylsalicylamide, niclosamide, osalmid, oxyclozanide, oxyphenbutazone, hydroxyprocaine p-aminosalicylate, phenetsal, phenyl salicylate, resorantel, salacetamide, salicyl, salicylanilide, salazosulfadimidine, salazosulfamide, salicylazosulfapyridine, 4-salicyloxymorphine, 4-sulfanilamidosalicylic acid, o-thymotic acid, 3',4',5'-trichlorosalicylanilide, xipamide, amidan, bromosaligenin and 3,5-diiodosalicylic acid;
(c) the mono- or polydehydroxylated residue of a phenolic tetracycline-type antibiotic selected from the group consisting of tetracycline, chlorotetracycline, demeclocycline, doxycycline, oxytetracycline, apicycline, clomocycline, guamecycline, methacycline, rolitetracycline, sancycline, lymecycline, mepicycline and mino cycline;
(d) the mono- or polydehydroxylated residue of a phenolic non-steroidal estrogen selected from the group consisting of benzestrol, dinestrol, diethylstilbestrol, hexestrol, mestilbol and promethestrol;
(e) the monodehydroxylated residue of a bio-affecting phenol selected from the group consisting of Vitamin $K_5$, 4-acetamino-2-methyl-1-naphthol, juglone, α-tocopherol, β-tocopherol, γ-tocopherol and δ-tocopherol;
(f) the mono- or polydehydroxylated residue of a phenolic narcotic analgesic or narcotic antagonist selected from the group consisting of apomorphine, hydromorphone, ketobemidone, morphine, nalorphine, naloxone, pentazocine, levorphanol, metopon, buprenorphine, butorphanol, cycloazocine, profadol, levallorphan, alazocine, nalbufine, oxilorphan, nalmexone, naltrexone and apocodeine, cyclorphan, desomorphine, dihydromorphine, 3-hydroxy-N-methylmorphinane, hydroxypethidine, levophenacylmorphan, metazocine, norlevophanol, normorphine, oxymorphine, phenazocine, phenomorphan, pholcodine and cyprenorphine;
(g) the monodehydroxylated residue of a bio-affecting phenol selected from the group consisting of chlorquinaldol, 5-chloro-8-quinolinol, clamoxyquin, 8-hydroxy-7-iodo-5-quinolinesulfonic acid, iodochlorhydroxyquin, octoquin, oxyquinoline, 8-hydroxyquinoline, chloroxine, amodioquin, diiodohydroxyquin and nitroxoline.

8. A compound as defined by Claim 1 wherein $R^2$ is selected from the group consisting of alkyl and aryl, and $R^3$ is selected from the group consisting of hydrogen, alkyl and aryl.

9. A pharmaceutical composition of matter comprising a sympathomimetically effective amount of a compound as defined by Claim 6 or 7(a) and a pharmaceutically acceptable carrier therefor.

10. A pharmaceutical composition of matter comprising an anti-inflammatory or analgesic effective amount of a compound as defined by Claim 7(b), and a pharmaceutically acceptable carrier therefor.

11. A pharmaceutical composition of matter comprising an antibiotic effective amount of a compound as defined by Claim 7(c), and a pharmaceutically acceptable carrier therefor.

12. A pharmaceutical composition of matter comprising an estrogenically effective amount of a compound as defined by Claim 7(d), and a pharmaceutically acceptable carrier therefor.

**Claims for the Contracting State: AT**

1. A process for preparing a compound selected from the group consisting of those having the structural formula (I):

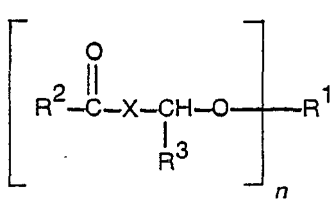

(I)

wherein

X is O, S or $NR^5$ wherein $R^5$ is hydrogen or lower alkyl;

$R^1$ is the nuclear mono- or polydehydroxylated residue of

1. a phenol selected from a group consisting of: hydroxyamphetamine, hydroxyamphetamine-

hydrobromide, metaraminol, metaraminol bitartrate, phenylephrine hydrochloride, oxymetazoline hydrochloride, albuterenol (albuterol), carbuterol hydrochloride, deterenol hydrochloride, quinterenol sulfate, soterenol, sulfonterol · hydrochloride, isoxuprine hydrochloride (isoxsuprine), nylidrin hydrochloride, bamethan, bamethan sulfate, mesuprine hydrochloride and ritodrine hydrochloride, benserazide, dimethophrine, *dl*-etilefrin, hordenine, p-hydroxyephedrine, ifenprodel, leptodactyline chloride, leptodactyline picrate, norfenfrine, octopamine, phentolamine, pholedrine, synephrine, tyramine and ethamivan;

2. a phenol which is a salicylic acid derivative or a structurally related compound selected from a group consisting of: acetaminophen, eugenol, p-aminosalicylic acid, 4-benzamidosalicylic acid, calcium benzoylpas, biphenamine, buclosamide, homosalate, hydroxyprocaine, methyl salicylate, oxybenzone, dioxybenzone, phenyl aminosalicylate, salicyl alcohol (saligenin), salicylamide, salicylic acid, salicylsalicylic acid (salsalate), sodium salicylate, difunisal, dibromsalan, fursalan, metabromsalan, bensalan, salethamide, and sacolex, p-aminosalicylic acid hydrazide, ammonium salicylate, 5-bromo-4'-chlorosalicylanilide, 5-bromosalicylhydroxamic acid, p-butylaminosalicylic acid 2-diethylaminoether ester, 3'-carboxy-4'-hydroxycinchophen, choline salicylate, 3,5--dibromosalicylaldehyde, fluorosalan, flopropione, gallacetophenone, gentisic acid, 4-hydroxyisophthalic acid, hydroxytetracaine, N-isopropylsalicylamide, niclosamide, osalmid, oxyclozanide, oxyphenbutazine, Pascaine® (hydroxyprocaine p-aminosalicylate), phenetsal, phenyl salicylate, resorantel, salacetamide, salicyl, salicylanilide, salazosulfadimidine, salazosulfamide, salicylazosulfapyridine, 4-salicyloxymorphine, 4-sulfanilamidosalicylic acid, o-thymotic acid, 3',4',5'-trichlorosalicylanilide, xipamide, amidan, bromosaligenin and 3,5-diiodosalicylic acid;

3. a non-steroidal estrogen phenol selected from a group consisting of: benzestrol, dinestrol, diethyl-stilbestrol, hexestrol, mestilbol and promesthestrol;

4. a phenol of tetracycline-type antibiotics selected from a group consisting of: tetracycline, tetracycline-hydrochloride, chlortetracycline, demeclocycline, doxycycline, oxytetracycline, apicycline, clomocycline, guamecycline, methacycline, rolitetracycline, rolitetracycline nitrate sesquihydrate, sancycline, lymecycline, mepicycline and minocycline;

5. phenols which are narcotic analgesics and narcotic antagonists selected from a group consisting of: morphine, apomorphine, hydromorphone hydrochloride, ketobemidone, morhine, morphine hydrochloride, morphine N-oxide, nalorphine hydrochloride, naloxone hydrochloride, pentazocine, levorphanol tartrate, metopon hydrochloride, butrenorphine hydrochloride, butorphanol, cyclazocine, profadol hydrochloride, levallorphan tartrate, alazocine, nalbufine hydrochloride, oxilorphan, nalmexone hydrochloride, naltrexone and apocodeine, cyclorphan, desomorphine, dihydromorphine, 3-hydroxy-N-methylmorphinane, hydroxypethidine, levophenacylmorphan, metazocine, norlevophanol, normorphine, oxymorphone, phenazocine, phenomorphan, pholcodine and cyprenorphine;

6. phenols which are anti-microbials including antibiotic/antibacterial, fungistatic, antimalarial and antiamebic agents selected from a group consisting of: 8-hydroxyquinoline, chlorquinaldol, 5-chloro-8-quinolinol, clamoxyquin, 8-hydroxy-7-iodo-5-quinolinesulfonic acid, iodochlorhydroxyquin, octoquin methylsulfate and oxyquinoline benzoate, rifamycin B, rifamycin SV, xanthocillin (X, $Y_1$ and $Y_2$) amoxicillin, celesticetin, chartreusin, novobiocin, resistomycin, rifamide, rifampin, albofungin, Formecin A, hygromycin, Acroteben®, amidan, antranorin, azosulfamide, diathymosulfone, Flavoteben, 1-isonicotinoyl-2-salicylidene hydrazide, lasalocid, myxin, siccanin, tyrocidine A, tyrocidine B, tyrocidine C and actinorhordine;

$R^2$ is selected from the group consisting of straight or branched chain alkyl having from 1 to 20 carbon atoms; aryl having from 6 to 10 carbon atoms; cycloalkyl having from 3 to 8 carbon atoms; alkenyl having from 2 to 20 carbon atoms; cycloalkenyl having from 4 to 8 carbon atoms; alkynyl having from 2 to 20 carbon atoms; aralkyl, alkaryl, aralkenyl, aralkynyl, alkenylaryl, alkynylaryl, (lower acyloxy)alkyl and carboxyalkyl, wherein alkyl, aryl, alkenyl and alkynyl are as defined above; 3- to 8-membered, saturated or unsaturated monoheterocyclic or polyheterocyclic containing from 1 to 3 of any one or more of the heteroatoms N, S or O; and mono- or polysubstituted derivatives of the above, each of the substituents being selected from the group consisting of lower alkyl, lower alkoxy, lower acyl, lower acyloxy, halo, halo(lower alkyl), cyano, carbethoxy, lower alkylthio, amino, nitro, lower alkylamino, di(lower alkyl)amino, carboxyl, carbamyl, lower alkylcarbamyl, di(lower alkyl)carbamyl and

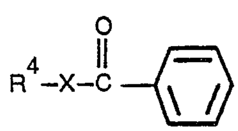

wherein $R^4$ is hydrogen or alkyl having from 1 to 10 carbons; $R^3$ is hydrogen, lower acyl, cyano, halo(lower alkyl), carbamyl, lower alkylcarbamyl, di(lower alkyl)carbamyl, $-CH_2ONO_2$, $-CH_2OCOR^2$, or any non-heterocyclic member of the group defined by $R^2$ above; and

*n* is at least one and equals the total number of phenolic hydroxyl functions comprising the $R^1$-phenol etherified via a $R^2COXCH(R^3)O-$ moiety; those having the structural formula (II):

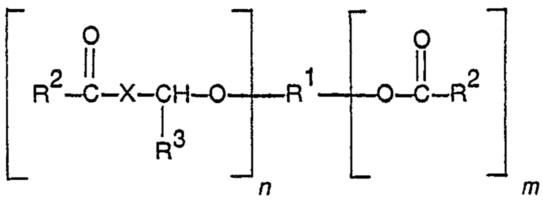

(II)

wherein X, $R^1$, $R^2$ and $R^3$ are as defined above, and $n$ and $m$ are each at least one and the sum of $n + m$ equals the total number of phenolic hydroxyl functions comprising the $R^1$-phenol either etherified via a $R^2COXCH(R^2)O$— moiety or esterified via a $R^2COO$— moiety; and the pharmaceutically acceptable acid addition salts, quaternary ammonium salts and N-oxides thereof, characterized in that

a) any functional groups except the nuclear hydroxy group of the parent phenol are suitably protected; the resulting derivative is reacted under $SN_2$ conditions with at least $n$ equivalents of a compound of the formula $R_2COXCH(R_3)Z$ wherein Z is a suitable leaving group, in the presence of at least n equivalents of a suitable base in a ketone solvent to form the corresponding derivative having $n$ acyl-X-methyl ether moieties in place of the $n$ nuclear hydroxy groups in the starting material; the protective groups are then removed with any common agent to form the desired compound,

b) to obtain the less substituted ethers the compound obtained in a) above is partially hydrolyzed or the process of a) above is carried out with less reactant $R_2COXCH(R_3)Z$,

c) to prepare the compounds of formula (II) the compounds of formula (I) which contain at least one free nuclear hydroxy group are reacted with a suitable acylating agent and

d) if desired the obtained compounds are transformed into the pharmaceutically acceptable addition salts, quaternary ammonium salts or N-oxides thereof.

2. Process as defined in Claim 1 for preparing a compound as defined by Claim 1, wherein the $n$ is 1, 2, 3 or 4.

3. Process as defined in Claim 1 for preparing a compound as defined by Claim 1, having the structural formula (II), wherein $n$ is I and $m$ is I.

4. Process as defined in Claim 1 for preparing a compound as defined by Claim 1, wherein $R^2$ is straight or branched chain alkyl having from 1 to 20 carbon atoms, aryl having from 6 to 10 carbon atoms, cycloalkyl having from 3 to 8 carbon atoms, alkenyl having from 2 to 20 carbon atoms, cycloalkenyl having from 4 to 8 carbon atoms or alkynyl having from 2 to 20 carbon atoms.

5. Process as defined in Claim 1 for preparing a compound as defined by Claim 1, wherein $R^3$ is hydrogen, alkyl, aryl or halo(lower alkyl).

6. Process as defined in Claim 1 for preparing a compound as defined by Claim 1, wherein $R^1$ is the monodehydroxylated residue of a nuclear monohydroxy sympathomimetic amine selected from the group consisting of hydroxyamphetamine, metaraminol and phenylephrine, oxymetazoline, albuterenol, carbuterol, deterenol, quinterenol, soterenol, sulfonterol, isoxuprine, nylidrin, bamethan, mesuprine and ritodrine.

7. Process as defined in Claim 1 for preparing a compound as defined by Claim 1, wherein $R^1$ is

(a) the mono- or polydehydroxylated residue of a nuclear mono- or trihydroxy sympathomimetic amine selected from the group consisting of benserazide, dimethophrine, *dl*-etilefrin, hordenine, p-hydroxyephedrine, ifenprodel, leptodactyline, norfenefrine, octapamine, phentolamine, pholedrine, synephrine, tyramine and ethamivan;

(b) the mono- or polydehydroxylated residue of a non-steroidal bio-affecting phenol selected from the group consisting of acetaminophen, eugenol, p-aminosalicylic acid, 4-benzamidosalicylic acid, calcium benzoylpas, biphenamine, buclosamide, homosalate, hydroxyprocaine, methyl salicylate, oxybenzone, dioxybenzone, phenyl aminosalicylate, salicyl alcohol, salicylamide, salicylic acid, salicylsalicylic acid, sodium salicylate, difunisal, octbenzone, salethamide, salcolex, p-aminosalicylic acid hydrazide, ammonium salicylate, 5-bromo-4′-chlorosalicylanilide, 5-bromosalicylhydroxamic acid, p-butylaminosalicylic acid 2-diethylaminoethyl ester, 3′-carboxy-4′-hydroxycinchopen, choline salicylate, 3,5-dibromosalicylaldehyde, flopropione, gallacetophenone, gentisic acid, 4-hydroxyisophthalic acid, hydroxytetracaine, N-isopropylsalicylamide, niclosamide, osalmid, oxyclozanide, oxyphenbutazone, hydroxyprocaine p-aminosalicylate, phenetsal, phenyl salicylate, resorantel, salacetamide, salicyl, salicylanilide, salazosulfadimidine, salazosulfamide, salicylazosulfapyridine, 4-salicyloxymorphine, 4-sulfanilamidosalicylic acid, o-thymotic acid, 3′,4′,5′-trichlorosalicylanilide, xipamide, amidan, bromosaligenin and 3,5-diiodosalicylic acid;

(c) the mono- or polydehydroxylated residue of a phenolic tetracycline-type antibiotic selected from the group consisting of tetracycline, chlorotetracycline, demeclocycline, doxycycline, oxytetracycline, apicycline, clomocycline, guamecycline, methacycline, rolitetracycline, sancycline, lymecycline, mepicycline and minocycline;

(d) the mono- or polydehydroxylated residue of a phenolic non-steroidal estrogen selected from the group consisting of benzestrol, dinestrol, diethylstilbestrol, hexestrol, mestilbol and promethestrol;

(e) the monodehydroxylated residue of a bio-affecting phenol selected from the group consisting of

Vitamin $K_5$, 4-acetamino-2-methyl-1-naphthol, juglone, α-tocopherol, β-tocopherol, γ-tocopherol and δ-tocopherol;

(f) the mono- or polydehydroxylated residue of a phenolic narcotic analgesic or narcotic antagonist selected from the group consisting of apomorphine, hydromorphone, ketobemidone, morphine, nalorphine, naloxone, pentazocine, levorphanol, metopon, buprenorphine, butorphanol, cycloazocine, profadol, levallorphan, alazocine, nalbufine, oxilorphan, nalmexone, naltrexone and apocodeine, cyclorphan, desomorphine, dihydromorphine, 3-hydroxy-N-methyl-morphinane, hydroxypethidine, levophenacylmorphan, metazocine, norlevophanol, normorphine, oxymorphone, phenazocine, phenomorphan, pholcodine and cyprenorphine;

(g) the monodehydroxylated residue of a bio-affecting phenol selected from the group consisting of chlorquinaldol, 5-chloro-8-quinolinol, clamoxyquin, 8-hydroxy-7-iodo-5-quinolinesulfonic acid, iodochlorhydroxyquin, octoquin, oxyquinoline, 8-hydroxyquinoline, chloroxine, amodioquin, diiodohydroxyquin and nitroxoline.

8. Process as defined in Claim 1 for preparing a compound as defined in Claim 1, wherein $R^2$ is selected from the group consisting of alkyl and aryl, and $R^3$ is selected from the group consisting of hydrogen, alkyl and aryl.

9. Process as defined in Claim 1 for preparing a compound as defined by Claim 1, which is N-phenoxy-methylbenzamide, N-(4-acetamidophenoxymethyl)benzamide, α-(N-methyl)aminomethyl-3-(pivaloyloxy-methoxy)benzyl alcohol, α-pivaloyloxyanisole, 3,4-bis(pivaloyloxymethoxy)benzaldehyde, 3-pivaloyloxy-4-pivaloyloxymethoxybenzaldehyde.

**Patentansprüche für die Vertragsstaaten: BE CH DE FR GB IT LI LU NL SE**

1. Eine Verbindung, ausgewählt aus der Gruppe, die aus solchen mit der Strukturformel (I):

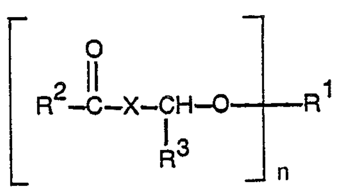 , (I)

worin

X O, S oder $NR^5$ ist, wobei $R^5$ Wasserstoff oder Niedrigalkyl ist;

$R^1$ der Kern-mono- oder -polyenthydroxylierte Rest von

1. einem Phenol, ausgewählt aus einer aus: Hydroxyamphetamin, Hydroxyamphetamin-hydrobromid, Metaraminol, Metaraminolbitartrat, Phenylephrinhydrochlorid, Oxymetazolinhydrochlorid, Albuterenol (Albuterol), Carbuterolhydrochlorid, Deterenolhydrochlorid, Quinterenolsulfat, Soterenol, Sulfonterolhydrochlorid, Isoxuprinhydrochlorid (Isoxsuprin), Nylidrinhydrochlorid, Bamethan, Bamethensulfat, Mesuprinhydrochlorid und Ritodrinhydrochlorid, Benserazid, Dimethophrin, dl-Etilefrin, Hordenin, p-Hydroxyephedrin, Ifenprodel, Leptodactylinchlorid, Leptodactylinpicrat, Norfenefrin, Octopamin, Phentolamin, Pholedrin, Synephrin, Tyramin und Ethamivan bestehenden Gruppe;

2. einem Phenol, das ein Salicylsäurederivat oder eine strukturell verwandte Verbindung, ausgewählt aus einer aus: Acetaminophen, Eugenol, p-Aminosalicylsäure, 4-Benzamidosalicylsäure, Calciumbenzoylpas, Biphenamin, Buclosmid, Homosalat, Hydroxyprocain, Methylsalicylat, Oxybenzon, Dioxybenzon, Phenylaminosalicylat, Salicylalkohol (Saligenin), Salicylamid, Salicylsäure,a Salicylsalicylsäure (Salsalat), Natriumsalicylat, Diflunisal, Dibromsalan, Fursalan, Metabromsalan, Bensalan, Salethamid und Salcolex, p-Aminosalicylsäurehydrazid, Ammoniumsalicylat, 5-Brom-4'-chlorsalicylanilid, 5-Bromsalicylhydroxamsäure, p-Butylaminosalicylsäure-2-diethylaminoethylester, 3'-Carboxy-4'hydroxycinchophen, Cholinsalicylat, 3,5-Dibromsalicylaldehyd, Fluorosalan, Flopropion, Gallacetophenon, Gentisinsäure, 4-Hydroxyisophthalsäure, Hydroxytetracain, N-Isopropylsalicylamid, Niclosamid, Osalmid, Oxyclozanid, Oxyphenbutazon, Pascain® (Hydroxyprocain-p-aminosalicylat), Phenetsal, Phenylsalicylat, Resorantel, Salacetamid, Salicyl, Salicylanilid, Salazosulfadimidin, Salazosulfamid, Salicylazosulfapyridin, 4-Salicyloxymorphin, 4-Sulfanilamidosalicylsäure, o- Thymotinsäure, 3',4',5'-Trichlorsalicylanilid, Xipamid, Amidan, Bromsaligenin und 3,5-Diiodsalicylsäure bestehenden Gruppe, ist;

3. einem nicht-steroiden Östrogenphenol, ausgewählt aus einer aus: Benzestrol, Dinestrol, Diethyl-stilbestrol, Hexestrol, Mestilbol und Promethestrol bestehenden Gruppe;

4. einem Phenol von Tetracyclintypus-Antibiotika, ausgewählt aus einer aus: tetracyclin, Tetracyclinhydrochlorid, Chlortetracyclin, Demeclocyclin, Doxycyclin, Oxytetracyclin, Apicyclin, Clomocyclin, Guamecyclin, Methacyclin, Rolitetracyclin, Rolitetracyclinnitratsequihydrat Sancyclin, Lymecyclin, Mepicyclin und Minocyclin bestehenden Gruppe;

5. Phenolen, die narkotische Analgetika und narkotische Antagonisten sind, und die aus einer aus; Morphin, Apomorphin Hydromorphonhydrochlorid, Ketobemidon, Morhin, Morphinhydrochlorid, Morphin-N-oxid, Nalorphinhydrochlorid, Naloxonhydrochlorid, Pentazocin, Levorphanoltartrat, Metoponhydrochlorid, Buprenorphinhydrochlorid, Butorphanol, Cyclazocin, Profadolhydrochlorid,

Levallorphantartrat, Alazocin, Nalbufinhydrochlorid, Oxilorphan, Nalmexonhydrochlorid, Naltrexon und Apocodein, Cyclorphan, Desomorphin, Dihydromorphin, 3-Hydroxy- N-methylmorphinan, Hydroxypethidin, Levophenacylmorphan, Metazocin, Norlevophanol, Normorphin, Oxymorphon, Phenazocin, Phenomorphan, Pholcodin und Cyprenorphin bestehenden gruppe ausgewählt sind;

6. Phenolen, die antimikrobielle Mittel sind, einschließlich antibiotisch/antibakterielle, fungistatische, antimalariawirksame und antiamöbenwirksame Mittel sind, welche aus einer aus; 8-Hydroxcychinolin, Chlorchinaldol, 5-Chlor-8-chinolinol, Clamoxyquin, 8-Hydroxy-7-iod-5-chinolinsulfonsäure, Iodchlorhydroxyquin, Octoquinmethylsulfat und Oxychinolinbenzoat, Rifamycin B, Rifamycin SV, Xanthocillin (X, $Y_1$ und $Y_2$) Amoxicillin, Celesticetin, Chartreusin, Novobiocin, Resistomycin, Rifamid, Rifampin, Albofungin, Formecin A, Hygromycin, Acroteben®, Amidan, Antranorin, Azosulfamid, Diathymosulfon, Flavoteben, 1-Isonicotinoyl-2-salicylidenhydrazid, Lasalocid, Myxin, Siccanin, Tyrocidin A, Tyrocidin B, Tyrocidin C und Actinorhordin bestehenden Gruppe ausgewählt sind, ist;

$R^2$ aus der Gruppe ausgewählt ist, die aus geradketigem oder verzweigtkettigem Alkyl mit 1 bis 20 Kohlenstoffatomen; Aryl mit 6 bis 10 Kohlenstoffatomen; Cyclkoalkyl mit 3 bis 8 Kohlenstoffatomen; Alkenyl mit 2 bis 20 Kohlenstoffatomen; Cycloalkenyl mit 4 bis 8 Kohlenstoffatomen; Alkinyl mit 2 bis 20 Kohlenstoffatomen; Aralkyl, Alkaryl, Aralkenyl, Aralkinyl, Alkenylaryl, Alkinylaryl, (Niedrigacylocy) alkyl und Carboxalkyl, wobei Alkyl, Aryl, Alkenyl und Alkinyl wie oben definiert sind; 3-bis-8-gliedrigen, gesättigten oder ungesättigten Monoheterocyclen oder Polyheterocyclen, enthaltend 1 bis 3 irgendeines oder mehrerer der Heteroatome N, S oder O; und mono- oder polysubstituierten Derivaten der obigen besteht, wobei jeder Substituenten aus der Gruppe ausgewählt ist, die aus Niedrigalkyl, Niedrigalkyloxy, Niedrigacyl, Niedrigacyloxy, Halogen, Halogen(niedrigalkyl), Cyan, Carbethoxy, Niedrigalkylthio, Amino, Nitro, Niedrigalkylamino, Di(niedrigalkyl)amino, Carboxyl, Carbamyl, Niedrigalkycarbamyl, Di(niedrigalkyl)carbamyl und

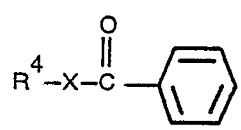

besteht, wobei $R_4$ Wasserstoff oder Alkyl mit 1 bis 10 Kohlenstoffatomen ist; $R_3$ Wasserstoff, Niedrigacyl, Cyan, Halogen(niedrigalkyl), Carbamyl, Niedrigalkylcarbamyl, Di(niedrigalkyl)carbamyl, $-CH_2ONO_2$, $-CH_2OCOR^2$, oder irgendein nicht-heterocyclisches Glied der durch $R^2$ oben definierten Gruppe ist; und

n wenigstens 1 ist, und der Gesamtzahl von phenolischen Hydroxylfunktionen, welche das R'-Phenol über einen $R^2COXCH(R^3)O$-Rest verethert aufweisen, entspricht; und aus solchen mit der Strukturformel (II):

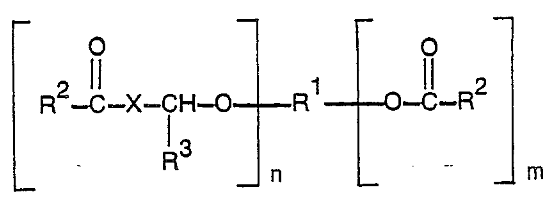

(II)

besteht, in der X, $R^1$, $R^2$ und $R^3$ wie oben definiert sind, und n und m jeweils wengistens 1 sind, und die Summe von n + m der Gesamtzahl von phenolischen Hydroxylfunktionen entspricht, welche das $R^1$-Phenol entweder über einen $R^2COXCH(R^3)O$-Rest verethert oder über einen $R^2COO$-Rest verestert aufweisen; und die pharmazeutisch annehmbaren Säureadditionssalze, quarternären Ammoniumsalze und N-Oxide davon.

2. Eine Verbindung, wie durch Anspruch 1 definiert, worin n 1, 2, 3 oder 4 ist.

3. Eine Verbindung, wie durch Anspruch 1 definiert, die die Strukturformel (II) aufweist, worin n 1 ist und m 1 ist.

4. Eine Verbindung wie in Anspruch 1 definiert, worin $R^2$ geradkettiges oder verzweigtkettiges Alkyl mit 1 bis 20 Kohlenstoffatomen, Aryl mit 6 bis 10 Kolenstoffatomen, Cycloalkyl mit 3 bis 8 Kohlenstoffatomen, Alkenyl mit 2 bis 20 Kohlenstoffatomen, Cycloalkenyl mit 4 bis 8 Kohlenstoffatomen oder Alkinyl mit 2 bis 20 Kohlenstoffatomen ist.

5. Eine Verbindung, wie durch Anspruch 1 definiert, worin $R_3$ Wasserstoff, Alkyl, Aryl oder Halogen(niedrigalkyl) ist.

6. Eine Verbindung, wie durch Anspruch 1 definiert, worin $R^1$ der monoenthydroxylierte Rest eines Kern-monohydroxysympathomimetischen Amins ist, das aus der aus Hydroxyamphetamin, Metaraminol und Phenylephrin, Oxymetazolin, Albuterenol, Carbuterol, Deterenol, Quinterenol, Soterenol, Sulfonterol, Isoxuprin, Nylidrin, Bamethan, Mesuprin und Ritodrin bestehenden Gruppe ausgewählt ist.

7. Eine Verbindung, wie durch Anspruch 1 defintert, worin $R^1$

(a) der mono- oder polyenthydroxylierte Rest eines Kern-mono- oder -trihydroxysympathomimetischen Amins, ausgewählt aus der aus Benserazid, Dimethophrin, dl-Etilefrin, Hordenin, p-Hydroxyephedrin, Ifenprodel, Leptodactylin, Norfenefrin, Octapamin, Phentolamin, Pholedrin, Synephrin. Tyramin und Ethamivan bestehenden Gruppe;

(b) der mono- oder -polyenthydroxylierte Rest eines nicht-steroiden, biowirksamen Phenols, ausgewählt aus der aus Acetaminophen, Eugenol, p-Aminosalicylsäure, 4-Benzamidosalicylsäure, Calciumbenzoylpas, Biphenamin, Buclosamid, Homosalat, Hydroxyprocain, Methylsalicylat, Oxybenzon, Dioxybenzon, Phenylaminosalicylat, Salicylalkohol, Salicylamid, Salicylsäure, Salicylsalicylsäure, Natriumsalicylat, Diflunisal, Octbenzon, Salethamid, Salcolex, p-Aminosalicylsäurehydrazid, Ammoniumsalicylat, 5-Brom-4'-chlorsalicylanilid, 5-Bromsalicylhydroxamsäure, p-Butylaminosalicylsäure-2-diethylaminoethylester, 3'-Carboxy-4'-hydroxycinchopen, Cholinsalicylat, 3,5-Dibromsalicylaldehyd, Flopropin, Gallacetophenon, Gentisinsäure, 4-Hydroxyisophthalsäure, Hydroxytetracain, N-Isopropylsalicylamid, Niclosamid, Osalmid, Oxyclozanid, Oxyphenbutazon, Hydroxyprocain-p-aminosalicylat, Phenetsal, Phenylsalicylat, Resorantel, Salacetamid, Salicyl, Salicylanilid, Salazosulfadimidin, Salazosulfamid, Salicylazosulfapyridin, 4-Salicyloxymorphin, 4-Sulfanilamidosalicylsäure, o-Thymotinsäure, 3',4',5'-Trichlorsalicylanilid, Xipamid, Amidan, Bromsaligenin und 3,5-Diiodsalicylsäure bestehenden Gruppe;

(c) der mono- oder polyenthydroxylierte Rest eines phenolischen Tetracyclintypus-Antibiotikums, ausgewählt aus der aus Tetracylin, Chlortetracylin, Demeclocyclin, Doxycyclin, Oxytetracyclin, Apicyclin, Clomocyclin, Guamecyclin, Methacyclin, Rolitetracyclin, Sancyclin, Lymecyclin, Mepicyclin, und Minocyclin bestehenden Gruppe;

(d) der mono- oder polyenthydroxylierte Rest eines phenolischen, nicht-steroiden Östrogens, ausgewählt aus der aus Benzestrol, Dinestrol, Diethylstilbestrol, Hexestrol, Mestilbol und Promethestrol bestehenden Gruppe;

(e) der monoenthydroxylierte Rest eines biowirksamen Phenols, ausgewählt aus der Vitamin $K_5$, 4-Acetamino-2-methyl-1-naphtol, Juglon, α-Tocopherol, β-Tocopherol, γ-Tocopherol und δ-Tocopherol bestehenden Gruppe;

(f) der mono- oder polyenthydroxylierte Rest eines phenolischen, narkotischen Analgetikums oder narkotischen Antagonisten, ausgewählt aus der aus Apomorphin, Hydromorphon, Ketobemidon, Morphin, Nalorphin, Naloxon, Pentazocin, Levorphanol, Metopon, Buprenorphin, Butorphanol, Cycloazocin, Profadol, Levallorphan, Alazocin, Nalbufin, Oxilorphan, Nalmexon, Naltrexon und Apocodein, Cyclorphan, Desomorphin, Dihydromorphin, 3-Hydroxy-N-methylmorphinan, Hydroxypethidin, Levophenacylmorphan, Metazocin, Norlevophanol, Normorphin, Oxymorphon, Phenazocin, Phenomorphan, Pholcodin und Cyprenorphin bestehenden Gruppe;

(g) der monoenthydroxylierte Rest eines biowirksamen Phenols, ausgewählt aus der aus Chlorchinaldol, 5-Chlor-8-chinolinol, Clamoxyquin, 8-Hydroxy-7-iod-5-chinolinsulfonsäure, Iodchlorhydroxyquin, Octoquin, Oxychinolin, 8-Hydroxychinolin, Chloroxin, Amodioquin, Diiodhydroxyquin und Nitroxolin bestehenden Gruppe ist.

8. Eine Verbindung, wie durch Anspruch 1 definiert, worin $R^2$ aus der aus Alkyl und Aryl bestehenden Gruppe ausgewählt ist, und $R^3$ aus der Gruppe ausgewählt ist, die aus Wasserstoff, Alkyl und Aryl besteht.

9. Eine pharamazeutische Zusammensetzung, enthaltend eine sympathomimetisch wirksame Menge einer Verbindung, wie durch Anspruch 6 oder 7 (a) definiert, und einen pharmazeutisch annehmbaren Träger dafür.

10. Eine pharmazeutische Zusammensetzung, enthaltend eine entzündungshemmende oder analgetisch wirksame Menge einer Verbindung, wie durch Anspruch 7 (b) definiert, und einen pharmazeutisch annehmbaren Träger dafür.

11. Eine pharmazeutische Zusammensetzung, enthaltend eine antibiotisch wirksame Menge einer Verbindung, wie durch Anspruch 7 (c) definiert, und einen pharmazeutisch annehmbaren Träger dafür.

12. Eine pharmazeutische Zusammensetzung, enthaltend eine östrogen wirksame Menge einer Verbindung, wie durch Anspruch 7 (d) definiert, und einen pharmazeutisch annehmbaren Träger dafür.

**Patentansprüche für den Vertragsstaat: AT**

1. Eine Verfahren zur Herstellung einer Verbindung, ausgewählt aus der Gruppe, die aus solchen mit der Strukturformel (I):

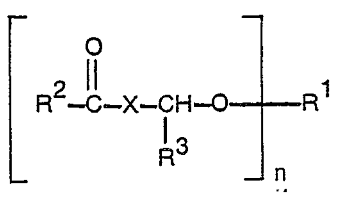 , (I)

worin

X O, S oder $NR^5$ ist, wobei $R^5$ Wasserstoff oder Niedrigalkyl ist;

$R^1$ der Kern-mono- oder -polyenthydroxylierte Rest von

1. einem Phenol, ausgewählt aus einer aus: Hydroxyamphetamin, Hydroxyamphetamin-hydrobromid, Metaraminol, Metaraminolbitartrat, Phenylephrinhydrochlorid, Oxymetazolinhydrochlorid, Albuterenol (Albuterol), Carbuterolhydrochlorid, Deterenolhydrochlorid, Quinterenolsulfat, Soterenol, Sulfonterolhydrochlorid, Isoxuprinhydrochlorid (Isoxsuprin), Nylidrinhydrochlorid, Bamethan, Bamethensulfat, Mesuprinhydrochlorid und Ritodrinhydrochlorid, Benserazid, Dimethophrin, dl-Etilefrin, Hordenin, p-Hydroxyephedrin, Ifenprodel, Leptodactylinchlorid, Leptodactylinpicrat, Norfenefrin, Octopamin, Phentolamin, Pholedrin, Synephrin, Tyramin und Ethamivan bestehenden Gruppe;

2. einem Phenol, das ein Salicylsäurederivat oder eine strukturell verwandte Verbindung, ausgewählt aus einer aus: Acetaminophen, Eugenol, p-Aminosalicylsäure, 4-Benzamidosalicylsäure, Calciumbenzoylpas, Biphenamin, Buclosamid, Homosalat, Hydroxyprocain, Methylsalicylat, Oxybenzon, Dioxybenzon, Phenylaminosalicylat, Salicylalkohol (Saligenin), Salicylamid, Salicylsäure, Salicylsalicylsäure (Salsalat), Natriumsalicylat, Diflunisal, Dibromsalan, Fursalan, Metabromsalan, Bensalan, Salethamid und Salcolex, p-Aminosalicylsäurehydrazid, Ammoniumsalicylat, 5-Brom-4'-chlorsalicylanilid, 5-Bromsalicylhydroxamsäure, p-Butylaminosalicylsäure-2-diethylaminoethylester, 3'-Carboxy-4'hydroxycinchophen, Cholinsalicylat, 3,5-Dibromsalicylaldehyd, Fluorosalan, Flopropion, Gallacetophenon, Gentisinsäure, 4-Hydroxyisophthalsäure, Hydroxytetracain, N-Isopropylsalicylamid, Niclosamid, Osalmid, Oxyclozanid, Oxyphenbutazon, Pascain® (Hydroxyprocain-p-aminosalicylat), Phenetsal, Phenylsalicylat, Resorantel, Salacetamid, Salicyl, Salicylanilid, Salazosulfadimidin, Salazosulfamid, Salicylazosulfapyridin, 4-Salicyloxymorphin, 4-Sulfanilamidosalicylsäure, o-Thymotinsäure, 3',4',5'-Trichlorsalicylanilid, Xipamid, Amidan, Bromsaligenin und 3,5-Diiodsalicylsäure bestehenden Gruppe, ist;

3. einem nicht-steroiden Östrogenphenol, ausgewählt aus einer aus: Benzestrol, Dinestrol, Diethyl-stilbestrol, Hexestrol, Mestilbol und Promethestrol bestehenden Gruppe;

4. einem Phenol von Tetracyclintypus-Antibiotika, ausgewählt aus einer aus: Tetracyclin, Tetracyclinhydrochlorid, Chlortetracyclin, Demeclocyclin, Doxycyclin, Oxytetracyclin, Apicyclin, Clomocyclin, Guamecyclin, Methacyclin, Rolitetracyclin, Rolitetracyclinnitratsequihydrat Sancyclin, Lymecyclin, Mepicyclin und Minocyclin bestehenden Gruppe;

5. Phenolen, die narkotische Analgetika und narkotische Antagonisten sind, und die aus einer aus; Morphin, Apomorphin Hydromorphonhydrochlorid, Ketobemidon, Morhin, Morphinhydrochlorid, Morphin-N-oxid, Nalorphinhydrochlorid, Naloxonhydrochlorid, Pentazocin, Levorphanoltartrat, Metoponhydrochlorid, Buprenorphinhydrochlorid, Butorphanol, Cyclazocin, Profadolhydrochlorid, Levallorphantartrat, Alazocin, Nalbufinhydrochlorid, Oxilorphan, Nalmexonhydrochlorid, Naltrexon und Apocodein, Cyclorphan, Desomorphin, Dihydromorphin, 3-Hydroxy- N-methylmorphinan, Hydroxypethidin, Levophenacylmorphan, Metazocin, Norlevophanol, Normorphin, Oxymorphon, Phenazocin, Phenomorphan, Pholcodin und Cyprenorphin bestehenden gruppe ausgewählt sind;

6. Phenolen, die antimikrobielle Mittel sind, einschließlich antibiotisch/antibakterielle, fungistatische, antimalariawirksame und antiamöbenwirksame Mittel sind, welche aus einer aus; 8-Hydroxychinolin, Chlorchinaldol, 5-Chlor-8-chinolinol, Clamoxyquin, 8-Hydroxy-7-iod-5-chinolinsulfonsäure, lodchlorhydroxyquin, Octoquinmethylsulfat und Oxychinolinbenzoat, Rifamycin B, Rifamycin SV, Xanthocillin (X, $Y_1$ und $Y_2$) Amoxicillin, Celesticetin, Chartreusin, Novobiocin, Resistomycin, Rifamid, Rifampin, Albofungin, Formecin A, Hygromycin, Acroteben®, Amidan, Antranorin, Azosulfamid, Diathymosulfon, Flavoteben, 1-Isonicotinoyl-2-salicylidenhydrazid, Lasalocid, Myxin, Siccanin, Tyrocidin A, Tyrocidin B, Tyrocidin C und Actinorhordin bestehenden Gruppe ausgewählt sind, ist;

$R^2$ aus der Gruppe ausgewählt ist, die aus geradketigem oder verzweigtkettigem Alkyl mit 1 bis 20 Kohlenstoffatomen; Aryl mit 6 bis 10 Kohlenstoffatomen; Cyclkoalkyl mit 3 bis 8 Kohlenstoffatomen; Alkenyl mit 2 bis 20 Kohlenstoffatomen; Cycloalkenyl mit 4 bis 8 Kohlenstoffatomen; Alkinyl mit 2 bis 20 Kohlenstoffatomen, Aralkyl, Alkaryl, Aralkenyl, Aralkinyl, Alkenylaryl, Alkinylaryl, (Niedrigacyloxy)alkyl und Carboxalkyl, wobei Alkyl, Aryl, Alkenyl und Alkinyl wie oben definiert sind; 3- bis 8-gliedrigen, gesättigten oder ungesättigten Monoheterocyclen oder Polyheterocyclen, enthaltend 1 bis 3 irgendeines oder mehrerer der Heteroatome N, S oder O; und mono- oder polysubstituierten Derivaten der obigen besteht, wobei jeder der Substituenten aus der Gruppe ausgewählt ist, die aus Niedrigalkyl, Niedrigalkoxy, Niedrigacyl, Niedrigacyloxy, Halogen, Halogen(niedrigalkyl), Cyan, Carbethoxy, Niedrigalkylthio, Amino, Nitro, Niedrigalkylamino, Di(niedrigalkyl)amino, Carboxyl, Carbamyl, Niedrigalkycarbamyl, Di(niedrig-alkyl)carbamyl und

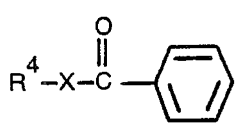

besteht, wobei $R^4$ Wasserstoff oder Alkyl mit 1 bis 10 Kohlenstoffatomen ist; $R^3$ Wasserstoff, Niedrigacyl, Cyan, Halogen(niedrigalkyl), Carbamyl, Niedrigalkylcarbamyl, Di(niedrigalkyl)carbamyl, $-CH_2ONO_2$, $-CH_3OCOR^2$ oder irgendein nicht-heterocyclisches Glied der durch $R^2$ oben definierten Gruppe ist; und

n wenigstens 1 ist, und der Gesamtzahl von phenolischen Hydroxylfunktionen, welche das $R^1$-Phenol über einen $R^2COXCH(R^3)O$-Rest verethert aufweisen, entspricht; und aus solchen mit der Strukturformel (II):

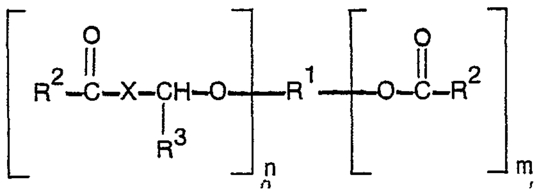

(II)

besteht, in der X, $R^1$, $R^2$ und $R^3$ wie oben definiert sind, und n und m jeweils wengistens 1 sind, und die Summe von n + m der Gesamtzahl von phenolischen Hydroxylfunktionen entspricht, welche das $R^1$-Phenol entweder über einen $R^2COXCH(R^3)O$—Rest verethert oder über einen $R^2COO$-Rest verestert aufweisen; und die pharmazeutisch annehmbaren Säureadditionssalze, quarternären Ammoniumsalze und N-Oxide davon, dadurch gekennzeichnet, daß man

a) irgendwelche funktionellen Gruppen, ausgenommen die Kernhydroxygruppe des Stammphenols, in geeigneter Weise schützt; das entstandene Derivat unter $SN_2$-Bedingungen mit wenigstens n Äquivalenten einer Verbindung der formel $R_2COXCH(R_3)Z$, worin Z eine geeignete austretende Gruppe ist, in Gegenwart von wenigstens n Äquivalenten einer geeigneten Base in einem Ketonlösungsmittel unter Bildung des entsprechenden Derivats das n Acyl-X-methyletherreste anstelle der im Ausgangsmaterial vorliegenden n Kernhydroxygruppen aufweist, umsetzt; und die Schutzgruppen dann mit irgendeinem üblichen Mittel unter Bildung der gewünschten Verbindung entfernt,

b) zur Gegenwart der in geringerem Maße substituierten Ether die gemäß a) oben erhaltene Verbindung partiell hydrolysiert oder das Verfahren gemäß a) oben mit weniger an Reaktionsteilnehmer $R_2COXCH(R_3)Z$ ausführt,

c) zur Herstellung der Verbindungen der Formel (II) die Verbindungen der Formel (I), welche wenigstens eine freie Kernhydroxygruppe enthalten, mit einem geeigneten Acylierungsmittel umsetzt, und

d) gewünschtenfalls die erhaltenen Verbindungen in die pharmazeutisch annehmbaren Additionssalze, quaternären Ammoniumsalze oder N-Oxide davon umwandelt.

2. Verfahren wie in Anspruch 1 definiert, zur Herstellung einer Verbindung wie durch Anspruch 1 definiert, worin n 1, 2, 3 oder 4 ist.

3. Verfahren wie in Anspruch 1 definiert, zur Herstellung einer Verbindung, wie durch Anspruch 1 definiert, die die Strukturformel (II) aufweist, worin n 1 ist und m 1 ist.

4. Verfahren wie in Anspruch 1 definiert, zur Herstellung einer Verbindung wie durch Anspruch 1 definiert, worin $R^2$ geradkettiges oder verzweigtkettiges Alkyl mit 1 bis 20 Kohlenstoffatomen, Aryl mit 6 bis 10 Kolenstoffatomen, Cycloalkyl mit 3 bis 8 Kohlenstoffatomen, Alkenyl mit 2 bis 20 Kolenstoffatomen, Cycloalkenyl mit 4 bis 8 Kohlenstoffatomen oder Alkinyl mit 2 bis 20 Kohlenstoffatomen ist.

5. Verfahren wie in Anspruch 1 definiert, zur Herstellung einer Verbindung, wie durch Anspruch 1 definiert, worin $R^3$ Wasserstoff, Alkyl, Aryl oder Halogen(niedrigalkyl) ist.

6. Verfahren wie in Anspruch 1 definiert, zur Herstellung einer Verbindung, wie durch Anspruch 1 definiert, worin $R^1$ der monoenthydroxylierte Rest eines Kern-monohydroxysympathomimetischen Amins ist, das aus der aus Hydroxyamphetamin, Metaraminol und Phenylephrin, Oxymetazolin, Albuterenol, Carbuterol, Deterenol, Quinterenol, Soterenol, Sulfonterol, Isoxuprin, Nylidrin, Bamethan, Mesuprin und Ritodrin bestehenden Gruppe ausgewählt ist.

7. Verfahren wie in Anspruch 1 definiert, zur Herstellung einer Verbindung, wie durch Anspruch 1 definiert, worin $R^1$

(a) der mono- oder polyenthydroxylierte Rest eines Kern-mono- oder -trihydroxysympathomimetischen Amins, ausgewählt aus der aus Benserazid, Dimethophrin, dl-Etilefrin, Hordenin, p-Hydroxyephedrin, Ifenprodel, Leptodactylin, Norfenefrin, Octapamin, Phentolamin, Pholedrin, Synephrin. Tyramin und Ethamivan bestehende Gruppe;

(b) der mono- oder -polyenthydroxylierte Rest eines nicht-steroiden, biowirksamen Phenols, ausgewählt aus der aus Acetaminophen, Eugenol, p-Aminosalicylsäure, 4-Benzamidosalicylsäure, Calciumbenzoylpas, Biphenamin, Buclosamid, Homosalat, Hydroxyprocain, Methylsalicylat, Oxybenzon, Dioxybenzon, Phenylaminosalicylat, Salicylalkohol, Salicylamid, Salicylsäure, Salicylsalicylsäure, Natriumsalicylat, Diflunisal, Octbenzon, Salethamid, Salcolex, p-Aminosalicylsäurehydrazid, Ammoniumsalicylat, 5-Brom-4'-chlorsalicylanilid, 5-Bromsalicylhydroxamsäure, p-Butylaminosalicylsäure-2-diethylaminoethylester, 3'-Carboxy-4'-hydroxycinchopen, Cholinsalicylat, 3,5-Dibromsalicylaldehyd, Flopropin, Gallacetophenon, Gentisinsäure, 4-Hydroxyisophthalsäure, Hydroxytetracain, N-Isopropylsalicylamid, Niclosamid, Osalmid, Oxyclozanid, Oxyphenbutazon, Hydroxyprocain-p-aminosalicylat, Phenetsal, Phenylsalicylat, Resorantel, Salacetamid, Salicyl, Salicylanilid, Salazosulfadimidin, Salazosulfamid, Salicylazosulfapyridin, 4-Salicyloxymorphin, 4-Sulfanilamidosalicylsäure, o-Thymotinsäure, 3',4',5'-Trichlorsalicylanilid, Xipamid, Amidan, Bromsaligenin und 3,5-Diiodsalicylsäure bestehenden Gruppe;

(c) der mono- oder polyenthydroxylierte Rest eines phenolischen Tetracyclintypus-Antibiotikums, ausgewählt aus der aus Tetracylin, Chlortetracylin, Demeclocyclin, Doxycyclin, Oxytetracyclin, Apicyclin, Clomocyclin, Guamecyclin, Methacyclin, Rolitetracyclin, Sancyclin, Lymecyclin, Mepicyclin, und Minocyclin bestehenden Gruppe;

(d) der mono- oder polyenthydroxylierte Rest eines phenolischen, nicht-steroiden Östrogens,

ausgewählt aus der aus Benzestrol, Dinestrol, Diethylstilbestrol, Hexestrol, Mestilbol und Promethestrol bestehenden Gruppe;

(e) der monoenthydroxylierte Rest eines biowirksamen Phenols, ausgewählt aus der Vitamin $K_5$, 4-Acetamino-2-methyl-1-naphtol, Juglon, α-Tocopherol, β-Tocopherol, γ-Tocopherol und δ-Tocopherol bestehenden Gruppe;

(f) der mono- oder polyenthydroxylierte Rest eines phenolischen, narkotischen Analgetikums oder narkotischen Antagonisten, ausgewählt aus der aus Apomorphin, Hydromorphon, Ketobemidon, Morphin, Nalorphin, Naloxon, Pentazocin, Levorphanol, Metopon, Buprenorphin, Butorphanol, Cycloazocin, Profadol, Levallorphan, Alazocin, Nalbufrin, Oxilorphan, Nalmexon, Naltrexon und Apocodein, Cyclorphan, Desomorphin, Dihydromorphin, 3-Hydroxy-N-methylmorphinan, Hydroxypethidin, Levophenacylmorphan, Metazocin, Norlevophanol, Normorphin, Oxymorphon, Phenazocin, Phenomorphan, Pholcodin und Cyprenorphin bestehenden Gruppe;

(g) der monoenthydroxylierte Rest eines biowirksamen Phenols, ausgewählt aus der aus Chlorchinaldol, 5-Chlor-8-chinolinol, Clamoxyquin, 8-Hydroxy-7-iod-5-chinolinsulfonsäure, Iodchlorhydroxyquin, Octoquin, Oxychinolin, 8-Hydroxychinolin, Chloroxin, Amodioquin, Diiodhydroxyquin und Nitroxolin bestehenden Gruppe ist.

8. Verfahren wie in Anspruch 1 definiert, zur Herstellung einer Verbindung, wie durch Anspruch 1 definiert, worin $R^2$ aus der aus Alkyl und Aryl bestehenden Gruppe ausgewählt ist, und $R^3$ aus der Gruppe ausgewählt ist, die aus Wassertoff, Alkyl und Aryl besteht.

9. Verfahren wie in Anspruch 1 definiert, zur Herstellung einer Verbindung, wie durch Anspruch 1 definiert, welche Verbindung N-Phenoxymethylbenzamid, N-(4-Acetamidophenoxymethyl)benzamid, α-(N-Methyl)aminomethyl-3-(pivaloyloxymethoxy)benzylalkohol, α-Pivaloyloxyanisol, 3,4-Bis(pivaloyloxymethoxy)benzaldehyd oder 3-Pivaloyloxy-4-pivaloyloxymethoxybenzaldehyd ist.

**Revendications pour les Etats contractants: BE CH DE FR GB IT LI LU NL SE**

1. Composé choisi dans le groupe constitué par ceux qui ont la formule développée (I):

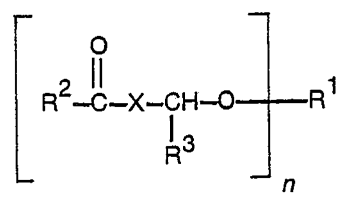

(I)

où

X est O, S ou $NR^5$ où $R^5$ est un hydrogène ou un alcoyle inférieur;

$R^1$ est le résidu nucléaire mono- ou polydéshydroxylé de

1. un phénol choisi dans un groupe constitué par: hydroxyamphétamine, bromhydrate d'hydroxyamphétamine, métaraminol, bitartrate de métaraminol, chlorhydrate de phényléphrine, chlorhydrate d'oxymétazoline, albuterénol (albutérol), chlorhydrate de carbutérol, chlorhydrate de détérénol, sulfate de quintérénol, sotérénol, chlorhydrate de sulfontérol chlorhydrate d'isoxuprine (isoxsuprine), chlorhydrate de nylidrine, baméthan, sulfate de baméthan, chlorhydrate de mésuprine et chlorhydrate de ritodrine, bensérazide, diméthophrine, *dl*-étiléfrine, hordénine, p-hydroxyéphédrine, ifenprodel, chlorure de leptodactyline, picrate de leptodactyline, norfénéfrine, octopamine, phentolamine, pholédrine, synéphrine, tyramine, et éthamivan;

2. un phénol qui est un dérivé d'acide salicylique ou un composé structurellement apparenté choisi dans un groupe constitué par: acétaminophène, eugénol, acide p-aminosalicylique, acide 4-benzamidosalicyclique, benzoylpas calcique, biphénamine, buclosamide, homosalate, hydroxyprocaïne, salicylate de méthyle, oxybenzone, dioxybenzone, aminosalicylate de phényle, alcool salicylique (saligénine), salicylamide, acide salicylique, acide salicylsalicylique (salsalate), salicylate de sodium, diflunisal, dibromosalan, fursalan, métabromosalan, bensalan, saléthamide, et salcolex, hydrazide de l'acide p-aminosalicylique, salicylate d'ammonium, 5-bromo-4'-chlorsalicylanilide, acide 5-bromosalicylhydroxamique, 2-diéthylaminoéthyl-ester de l'acide p-butyl-aminosalicylique, 3'-carboxy-4'-hydroxycinchophène, salicylate de choline, 3,5-dibromosalicylaldéhyde, fluorosalan, flopropione, gallacétophénone, acide gentisique, acide 4-hydroxyisophtalique, hydroxytétracaïne, N-isopropylsalicylamide, niclosamide, osalmide, oxyclozanide, oxyphénbutazone, Pascaïne® (p-aminosalicylate d'hydroxyprocaïne, procaïne), phénétsal, salicylate de phényle, résorantel, salacétamide, salicyle, salcylanilide, salazosulfadimidine, salazosulfamide, salicylazosulfapuridine, 4-salicyloxymorphine, acide 4-sulfanilamidosalicylique, acide o-thymotique, 3',4',5'-trichlorosalicylanilide, xipamide, amidan, bromosaligénine et acide 3,5-diiodosalicylique;

3. un phénol oestrogène non stéroïdique choisi dans un groupe constitué par: benzestrol, dinestrol, diéthylstilbestrol, hexestrol, mestilbol et prométhestrol;

4. un phénol d'antibiotiques du type tétracycline choisi dans un groupe constitué par: tétracycline, chlorhydrate de tétracycline, chlorotétracycline, déméclocycline, doxycycline, oxytétracycline, apicycline,

clomocycline, guamécycline, méthacycline, rolitétracycline, nitrate de rolitétracycline sesquihydraté, sancycline, lymécycline, mépicycline et minocycline;

5. les phénols qui sont des analgésiques narcotiques et de antagonistes narcotiques choisis dans un groupe constitué par: morphine, apomorphine, chlorhydrate d'hydromorphone, cétobémidone, morhine, chlorhydrate de morphine, N-oxyde de morphine, chlorhydrate de nalorphine, chlorhydrate de naloxone, pentazocine, tartrate de lévorphanol, chlorhydrate de métopon, chlorhydrate de buprénorphine, butorphanol, cyclazocine, chlorhydrate de profadol, tartrate de levallorphan, alazocine, chlorhydrate de nalbufine, oxilorphan, chlorhydrate de nalmexone, naltrexone et apocodéine, cyclorphan, désomorphine, dihydromorphine, 3-hydroxy-N-méthyl-morphinane, hydroxypéthidine, lévophénacylmorphan, métazocine, norlévophanol, normorphine, oxymorphone, phénazocine, phénomorphan, pholcodine et cyprénorphine;

6. les phénols qui sont antimicrobiens y compris les agents antibiotiques/antibactériens, fongistatiques, antimalariens et antiamibiens choisis dans un groupe constitué par: 8-hydroxyquinoléine, chloroquinaldol, 5-chloro-8-quinolinol, clamoxyquine, acide 8-hydroxy-7-iodo-5-quinoléinesulfonique, iodochlorohydroxyquine, méthylsulfate d'octoquine et benzoate d'oxyquinoléine, rifamycine B, rifamycine SV, xanthocilline (X, $Y^1$ et $Y^2$), amoxicilline, célesticétine, chartreusine, novobiocine, résistomycine, rifamide, rifampine, albofungine, Formécine A, hygromycine, Acroteben®, amidan, antranorine, azosulfamide, diathymosulfone, Flavoteben hydrazide de 1-isonicotinoyl-2-salicylidène, lasalocide, myxine, siccanine, tyrocidine A, tyrocidine B, tyrocidine C et actinorhordine; $R^2$ est choisi dans le groupe constitué par alcoyle à chaîne droite ou ramifiée ayant de 1 à 20 atomes de carbone; aryle ayant de 6 à 10 atomes de carbone; cycloalcoyle ayant de 3 à 8 atomes de carbone; alcényle ayant de 2 à 20 atomes de carbone; cycloalcényle ayant de 4 à 8 atomes de carbone; alcynyle ayant de 2 à 20 atomes de carbone; aralcoyle, alcaryle, aralcényle, aralcynyle, alcénylaryle, alcynylaryle, (acyloxy inférieur)alcoyle et carboxyalcoyle, où alcoyle, aryle, alcényle, et alcynyle sont tels que définis ci-dessus; corps monohétérocycliques ou polyhétérocycliques à 3 à 8 chaînons, saturés ou non saturés contenant de 1 à 3 d'un ou plusieurs des hétéroatomes N, S ou O; et les dérivés mono- ou polysubstitués de ce qui précède, chacun des substituants étant choisi dans le groupe constitué par alcoyle inférieur, alcoxy inférieur, acyl inférieur, acyloxy inférieur, halo, halo(alcoyle inférieur), cyano, carbéthoxy, alcoylthio inférieur, amino, nitro, alcoylamino inférieur, di(alcoyle inférieur)amino, carboxyle, carbamyle, alcoyle inférieur-carbamyle, di(alcoyle inférieur)carbamyle et

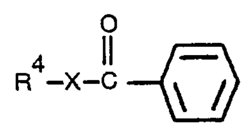

où $R^4$ est un hydrogène ou un alcoyle ayant de 1 à 10 atomes de carbone: $R^3$ est un hydrogène, acyle inférieur cyano, halo(alcoyle inférieur), carbamyle, alcoylcarbamyle inférieur, di(alcoyle inférieur)carbamyle, $—CH_2ONO_2$, $—CH_2OCOR^2$, ou tout membre non-hétérocyclique du groupe défini par $R^2$ ci-dessus; et

*n* vaut au moins un et ègale le nombre total de fonctions hydroxyle phénoliques comprenant le $R^1$-phénol éthérifié par une fraction $R^2COXCH(R^3)O—$; ceux de formule développée (II):

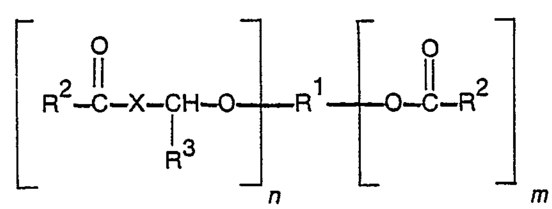

(II)

ou X, $R^1$, $R^2$ et $R^3$ sont tels que définis ci-dessus, et n et m valent chacun au moins un et la somme de $n+m$ égale le nombre total de fonctions hydroxyle phénoliques comprenant le $R^1$-phénol soit éthérifié par une fraction $R^2COXCH(R^3)O—$ soit estérifié par une fraction $R^2COO—$; et leurs sels d'addition d'acides, sels d'ammonium quaternaires et N-oxydes pharmaceutiquement acceptables

2. Composé tel que défini par la revendication 1 où *n* vaut 1, 2, 3 ou 4.

3. Composé tel que défini par la revendication 1, ayant la formule développée (II), où *n* vaut 1 et *m* vaut 1.

4. Composé tel que défini par la revendication 1, où $R^2$ est un alcoyle à chaîne droite ou ramifiée ayant de 1 à 20 atomes de carbone, un aryle ayant de 6 à 10 atomes de carbone, un cycloalcoyle ayant de 3 à 8 atomes de carbone, un alcényle ayant de 2 à 20 atomes de carbone, un cycloalcényle ayant de 4 à 8 atomes de carbone ou un alcynyle ayant de 2 à 20 atomes de carbone.

5. Composé tel que défini par la revendication 1, où $R^3$ est un hydrogène, alcoyle, aryle ou halo(alcoyle inférieur).

6. Composé tel que défini par la revendication 1, où $R^1$ est le résidu monodéshydroxylé d'une amine

sympathomimétique monohydroxy nucléaire choisi dans le groupe constitué par hydroxyamphétamine, métaraminol et phényléphrine, oxymétazoline, albutérénol, carbutérol, détérénol, quintérénol, sotérénol, sulfontérol, isoxuprine, nylidrine, baméthan, mésuprine et ritodrine.

7. Composé tel que défini la revendication 1, où $R^1$ est

(a) le résidu mono- ou polydéshydroxylé d'une amine sympathomimétique mono- ou trihydroxy nucléaire choisi dans le groupe constitué par benserazide, diméthophrine, *dl*-étiléfrine, hordénine, p-hydroxyéphédrine, ifenprodel, leptodactyline, norfénéfrine, octapamine, phentolamine, pholédrine, synéphrine, tyramine et éthamivan;

(b) le résidu mono- ou polydéshydroxylé d'un phénol bio-affectant non stéroïdique choisi dans le groupe constitué par acétaminophène, eugénol, acide p-aminosalicylique, acide 4-benzamidosalicylique, benzoylpas calcique, biphénamine, buclosamide, homosalate, hydroxyprocaïne, salicylate de méthyle, oxybenzone, dioxybenzone, aminosalicylate de phényle, alcool salicylique, salicylamide, acide salicylique, acide salicylsalicylique, salicylate de sodium, diflunisal, octbenzone, saléthamide, salcolex, hydrazide de l'acide p-aminosalicylique, salicylate d'ammonium, 5-bromo-4'-chlorosalicylanilide, acide 5-bromosalicylhydroxamique, 2-diéthylaminoéthylester de l'acide p-butylaminosalicylique, 3'-carboxy-4'-hydroxycinchophène, salicylate de choline, 3,5-dibromosalicylaldéhyde, flopropione, gallacétophénone, acide gentisique, acide 4-hydroxyisophtalique, hydroxytétracaïne, N-isopropylsalicylamide, niclosamide, osalmide, oxyclozanide, oxyphenbutazone, p-aminosalicylate d'hydroxyprocaïne, phénétsal, salicylate de phényle, résorantel, salacétamide, salicyle, salicylanilide, salazosulfadimidine, salazosulfamide, salicylazosulfapyridine, 4-salicyloxymorphine, acide 4-sulfanilamidosalicylique, acide o-thymotique, 3',4',5'-trichlorosalicylanilide, xipamide, amidan, bromosaligénine et acide 3,5-diiodosalicylique;

(c) le résidu mono- ou polydéshydroxylé d'un antibiotique phénolique du type tétracycline choisi dans le groupe constitué par tétracycline, chlorotétracycline, déméclocycline, doxycycline, oxytétracycline, apicycline, clomocycline, guamécycline, méthacycline, rolitétracycline, sancycline, lymécycline, mépicycline et minocycline;

(d) le résidu mono- ou polydéshydroxylé d'un oestrogène non stéroïdique phénolique choisi dans le groupe constitué par benzestrol, dinestrol, diéthylstilbestrol, hexestrol mestilbol et promethestrol;

(e) le résidu monodéshydroxylé d'un phénol bio-affectant choisi dans le groupe constitué par vitamine $K^5$, 4-acétamino-2-méthyl-1-naphtol, juglone, α-tocophérol, β-tocophérol, γ-tocophérol et δ-tocophérol;

(f) le résidu mono- ou polydéshydroxylé d'un analgésique narcotique ou antagoniste narcotique phénolique choisi dans le groupe constitué par apomorphine, hydromorphone, cétobémidone, morphine, nalorphine, naloxone, pentazocine, levorphanol, métopon, buprénorphine, butorphanol, cycloazocine, profadol, levallorphan, alazocine, nalbufine, oxilorphan, nalmexone, naltrexone et apocodéine, cyclorphan, désomorphine, dihydromorphine, 3-hydroxy-N-méthyl-morphinane, hydroxypéthidine, levophénacylmorphan, métazocine, norlevophanol, normorphine, oxymorphone, phénazocine, phénomorphan, pholcodine et cyprenorphine;

(g) le résidu monodéshydroxylé d'un phénol bio-affectant choisi dans le groupe constitué par chlorquinaldol, 5-chloro-8-quinolinol, clamoxyquine, acide 8-hydroxy-7-iodo-5-quinoléinesulfonique, iodochlorohydroxyquine, octoquine, oxyquinoléine, 8-hydroxy-quinoléine, chloroxine amodioquine, diiodohydroxyquine et nitroxoline.

8. Composé tel que défini dans la revendication 1, où $R^2$ est choisi dans le groupe constitué par alcoyle et aryle, et $R^3$ est choisi dans le groupe constitué par hydrogène, alcoyle et aryle.

9. Composition pharmaceutique de matière comprenant une quantité sympathomimétiquement efficace d'un composé tel que défini par les revendications 6 et 7 (a) et un support pharmaceutiquement acceptable à cet effet.

10. Composition pharmaceutique de matière comprenant une quantité antiinflammatoire ou analgésique efficace d'un composé tel que défini par la revendication 7 (b), et un support pharmaceutiquement acceptable à cet effet.

11. Composition pharmaceutique de matière comprenant une quantité antibiotique efficace d'un composé tel que dèfini par la revendication 7 (c), et un support pharmaceutiquement acceptable à cet effet.

12. Composition pharmaceutique de matière comprenant une quantité oestrogéniquement efficace d'un composé tel que dèfini par la revendication 7 (d) et un support pharmaceutiquement acceptable à cet effet.

**Revendications pour l'Etat contractant: AT**

1. Procédé de préparation d'un composè choisi dans le groupe constitué par ceux qui ont la formule développée (I):

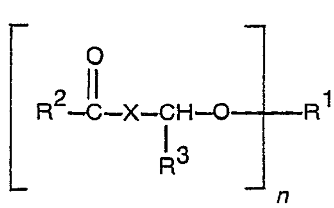

(I)

où

X est O, S ou $NR^5$ où $R^5$ est un hydrogène ou un alcoyle inférieur;

$R^1$ est le résidu nucléaire mono- ou polydéshydroxylé de

1. un phénol choisi dans un groupe constitué par: hydroxyamphétamine, bromhydrate d'hydroxyamphétamine, métaraminol, bitartrate de métaraminol, chlorhydrate de phényléphrine, chlorhydrate d'oxymétazoline, albutérénol (albutérol), chlorhydrate de carbutérol, chlorhydrate de détérénol, sulfate de quintérénol, sotérénol, chlorhydrate de sulfontérol chlorhydrate d'isoxuprine (isoxsuprine), chlorhydrate de nylidrine, baméthan, sulfate de baméthan, chlorhydrate de mésuprine et chlorhydrate de ritodrine, bensérazide, diméthophrine, *dl*-étiléfrine, hordénine, p-hydroxyéphédrine, ifenprodel, chlorure de leptodactyline, picrate de leptodactyline, norfénéfrine, octopamine, phentolamine, pholédrine, synéphrine, tyramine, et éthamivan;

2. un phénol qui est un dérivé d'acide salicylique ou un composé structurellement apparenté choisi dans un groupe constitué par: acétaminophène, eugénol, acide p-aminosalicylique, acide 4-benzamido-salicyclique, benzoylpas calcique, biphénamine, buclosamide, homosalate, hydroxyprocaîne, salicylate de méthyle, oxybenzone, dioxybenzone, aminosalicylate de phényle, alcool salicylique (saligénine), salicylamide, acide salicylique, acide salicylsalicylique (salsalate), salicylate de sodium, diflunisal, dibromosalan, fursalan, métabromosalan, bensalan, saléthamide, et salcolex, hydrazide de l'acide p-aminosalicylique, salicylate d'ammonium, 5-bromo-4'-chlorosalicylanilide, acide 5-bromosalicylhydroxamique, 2-diéthylaminoéthyl-ester de l'acide p-butyl-aminosalicylique, 3'-carboxy-4'-hydroxy-cinchophène, salicylate de choline, 3,5-dibromosalicylaldéhyde, fluorosalan, flopropione, gallacétophénone, acide gentisique, acide 4-hydroxyisophtalique, hydroxytétracaîne, N-isopropylsalicylamide, niclosamide, osalmide, oxyclozanide, oxyphénbutazone, Pascaîne® (p-aminosalicylate d'hydroxyprocaîne, procaîne), phénétsal, salicylate de phényle, résorantel, salacétamide, salicyle, salcylanilide, salazosulfadimidine, salazosulfamide, salicylazosulfapyridine, 4-salicyloxymorphine, acide 4-sulfanilamidosalicylique, acide o-thymotique, 3',4',5'-trichlorosalicylanilide, xipamide, amidan, bromosaligénine et acide 3,5-diiodosalicylique;

3. un phénol oestrogène non stéroîdique choisi dans un groupe constitué par: benzestrol, dinestrol, diéthylstilbestrol, hexestrol, mestilbol et prométhestrol;

4. un phénol d'antibiotiques du type tétracycline choisi dans un groupe constitué par: tétracycline, chlorhydrate de tétracycline, chlorotétracycline, déméclocycline, doxycycline, oxytétracycline, apicycline, clomocycline guamécycline, méthacycline, rolitétracycline, nitrate de rolitétracycline sesquihydraté, sancycline, lymécycline, mépicycline et minocycline;

5. les phénols qui sont des analgésiques narcotiques et de antagonistes narcotiques choisis dans un groupe constitué par: morphine, apomorphine, chlorhydrate d'hydromorphone, cétobémidone, morhine, chlorhydrate de morphine, N-oxyde de morphine, chlorhydrate de nalorphine, chlorhydrate de naloxone, pentazocine, tartrate de lévorphanol, chlorhydrate de métopon, chlorhydrate de buprénorphine, butorphanol, cyclazocine, chlorhydrate de profadol, tartrate de levallorphan, alazocine, chlorhydrate de nalbufine, oxilorphan, chlorhydrate de nalmexone, naltrexone et apocodéine, cyclorphan, désomorphine, dihydromorphine, 3-hydroxy-N-méthyl-morphinane, hydroxypéthidine, lévophénacylmorphan, métazocine, norlévophanol, normorphine, oxymorphone, phénazocine, phénomorphan, pholcodine et cyprénorphine;

6. les phénols qui sont antimicrobiens y compris les agents antibiotiques/antibactériens, fongistatiques, antimalariens et antiamibiens choisis dans un groupe constitué par: 8-hydroxyquinoléine, chloroquinaldol, 5-chloro-8-quinolinol, clamoxyquine, acide 8-hydroxy-7-iodo-5-quinoléinesulfonique, iodochlorohydroxyquine méthylsulfate d'octoquine et benzoate d'oxyquinoléine, rifamycine B, rifamycine SV, xanthocilline (X, $Y^1$ et $Y^2$), amoxicilline, célesticétine, chartreusine, novobiocine, résistomycine, rifamide, rifampine, albofungine, Formécine A, hygromycine, Acroteben ®, amidan, antranorine, azosulfamide, diathymosulfone, Flavoteben, hydrazide de 1-isonicotinoyl-2-salicylidène, lasalocide, myxine, siccanine, tyrocidine A, tyrocidine B, tyrocidine C et actinorhordine; $R^2$ est choisi dans le groupe constitué par alcoyle à chaîne droite ou ramifiée ayant de 1 à 20 atomes de carbone; aryle ayant de 6 à 10 atomes de carbone; cycloalcoyle ayant de 3 à 8 atomes de carbone; alcényle ayant de 2 à 20 atomes de carbone; cycloalcényle ayant de 4 à 8 atomes de carbone; alcynyle ayant de 2 à 20 atomes de carbone; aralcoyle, alcaryle, aralcényle, aralcynyle, alcénylaryle, alcynylaryle, (acyloxy inférieur)alcoyle et carboxyalcoyle, où alcoyle, aryle, alcényle, et alcynyle sont tels que définis ci-dessus; corps monohétérocycliques ou polyhétérocycliques à 3 à 8 chaînons, saturés ou non saturés contenant de 1 à 3 d'un ou plusieurs des hétéroatomes N, S ou O; et les dérivés mono- ou polysubstitués de ce qui précède, chacun des substituants étant choisi dans le groupe constitué par alcoyle inférieur, alcoxy inférieur, acyle inférieur, acyloxy inférieur, halo, halo(alcoyle inférieur), cyano, carbéthoxy, alcoylthio inférieur, amino, nitro, alcoylamino inférieur, di(alcoyle inférieur)amino, carboxyle, carbamyle, alcoyle inférieur-carbamyle, di(alcoyle inférieur)carbamyle et

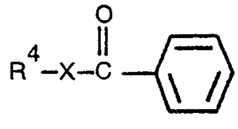

où $R^4$ est un hydrogène ou un alcoyle ayant de 1 à 10 atomes de carbone: $R^3$ est un hydrogène, acyle inférieur cyano, halo(alcoyle inférieur), carbamyle, alcoylcarbamyle inférieur, di(alcoyle inférieur)carbamyle, $—CH_2ONO_2$, $—CH_2OCOR^2$, ou tout membre non-hétérocyclique du groupe défini par $R^2$ ci-dessus; et

*n* vaut au moins un et ègale le nombre total de fonctions hydroxyle phénoliques comprenant le $R^1$-phénol éthérifié par une fraction $R^2COXCH(R^3)O—$; ceux de formule développée (II):

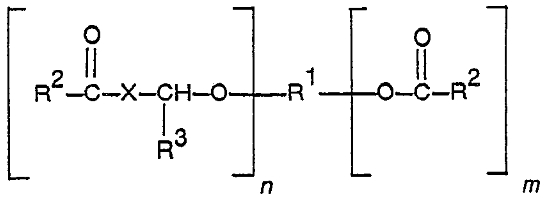

(II)

où X, $R^1$, $R^2$ et $R^3$ sont tels que définis ci-dessus, et n et m valent chacun au moins un et la somme de $n+m$ égale le nombre total de fonctions hydroxyle phénoliques comprenant le $R^1$-phénol soit éthérifié par une fraction $R^2COXCH(R^3)O—$ soit estérifié par une fraction $R^2COO—$; et leurs sels d'addition d'acides, sels d'ammonium quaternaires et N-oxydes pharmaceutiquement acceptables caractérisé en ce que

a) tous les groupes fonctionnels sauf le groupe hydroxy nucléaire du phénol générateur sont protégés de façon appropriée; le dérivé résultant est mis à réagir dans des conditions de $SN_2$ avec au moins *n* equivalents d'un composé de formule $R_2COXCH(R_3)Z$ où Z est un groupe sortant approprié, en présence d'au moins *n* équivalents d'une base appropriée dans un solvant cétone pour former le dérivé correspondant ayant *n* fractions acyl-X-méthyl-éther au lieu des *n* groupes hydroxy nucléaires dans le produit de départ; les groupes protecteurs sont alors enlevés avec n'importe quel agent courant pour former le composé désire,

b) pour obtenir les éthers moins substitués le composé obtenu en a) ci-dessus est partiellement hydrolysé ou le procédé de a) ci-dessus est partiellement hydrolysé ou le procédé de a) ci-dessus est conduit avec une moindre quantité de réactif $R_2COXCH(R_3)Z$,

c) pour préparer les composés de formule (II) on fait régir les composés de formule (I) qui contiennent au moins un groupe hydroxy nucléaire libre avec un agent acylant approrié et

d) si on le désire on transforme les composés obteunus en leurs sels d'addition sels d'ammonium quaternaires ou N-oxydes pharmaceutiquement acceptables.

2. Procédé selon la revendication 1 pour préparer un composé tel que défini par la revendication 1 où *n* vaut 1, 2, 3 ou 4.

3. Procédé tel que défini dans la revendication 1, pour préparer un composé tel que defini par la revendication 1, ayant la formule développée (II), où *n* vaut 1 et *m* vaut 1.

4. Procédé tel que défini dans la revendication 1, pour préparer un composé tel que défini par la revendication 1, où $R^2$ est un alcoyle à chaîne droite ou ramifiée ayant de 1 à 20 atomes de carbone, un aryle ayant de 6 à 10 atomes de carbone, un cycloalcoyle ayant de 3 à 8 atomes de carbone, un alcényle ayant de 2 à 20 atomes de carbone, un cycloalcényle ayant de 4 à 8 atomes de carbone ou un alcynyle ayant de 2 à 20 atomes de carbone.

5. Procédé tel que défini dans la revendication 1 pour préparer un composé tel que défini par la revendication 1, où $R^3$ est un hydrogène, alcoyle, aryle ou halo(alcoyle inférieur).

6. Procédé tel que défini dans la revendication 1 pour préparer un composé tel que défini par la revendication 1, où $R^1$ est le résidu monodéshydroxylé d'une amine sympathomimétique monohydroxy nucléaire choisi dans le groupe constitué par hydroxyamphétamine, métaraminol et phényléphrine, oxymétazoline, albutérénol, carbutérol, détérénol, quintérénol, sotérénol, sulfontérol, isoxuprine, nylidrine, baméthan, mésuprine et ritodrine.

7. Procédé tel que défini dans la revendication 1 pour préparer un composé tel que défini par la revendication 1, où $R^1$ est

(a) le résidu mono- ou polydéshydroxylé d'une amine sympathomimétique mono- ou trihydroxy nucléaire choisi dans le groupe constitué par benserazide, diméthophrine, *dl*-étiléfrine, hordénine, p-hydroxyéphédrine, ifenprodel, leptodactyline, norfénéfrine, octapamine, phentolamine, pholédrine, synéphrine, tyramine et éthamivan;

(b) le résidu mono- ou polydéshydroxylé d'un phénol bio-affectant non stéroïdique choisi dans le groupe constitué par acétaminophène, eugénol, acide p-aminosalicylique, acide 4-benzamidosalicylique, benzoylpas calcique, biphénamine, buclosamide, homosalate, hydroxyprocaîne, salicylate de méthyle, oxybenzone, dioxybenzone, aminosalicylate de phényle, alcool salicylique, salicylamide, acide salicylique, acide salicylsalicylique, salicylate de sodium, diflunisal, octbenzone, saléthamide, salcolex, hydrazide de l'acide p-aminosalicylique, salicylate d'ammonium, 5-bromo-4'-chlorosalicylanilide, acide 5-bromosalicyl-hydroxamique, 2-diéthylaminoéthylester de l'acide p-butylaminosalicylique, 3'-carboxy-4'-hydroxy-cinchophène, salicylate de choline, 3,5-dibromosalicylaldéhyde, flopropione, gallacétophénone, acide gentisique, acide 4-hydroxyisophtalique, hydroxytétracaîne, N-isopropylsalicylamide, niclosamide,

osalmide, oxyclozanide, oxyphebutazone, p-aminosalicylate d'hydroxyprocaïne, phénétsal, salicylate de phényle, résorantel, salacétamide, salicyle, salicylanilide, salazosulfadimidine, salazosulfamide, salicylazosulfapyridine, 4-salicyloxymorphine, acide 4-sulfanilamidosalicylique, acide o-thymotique, 3',4',5'-trichlorosalicylanilide, xipamide, amidan, bromosaligénine et acide 3,5-diiodosalicylique;

(c) le résidu mono- ou polydéshydroxylé d'un antibiotique phénolique du type tétracycline choisi dans le groupe constitué par tétracycline, chlorotétracycline, déméclocycline, doxycycline, oxytétracycline, apicycline, clomocycline, guamécycline, méthacycline, rolitétracycline, sancycline, lymécycline, mépicycline et minocycline;

(d) le résidu mono- ou polydéshydroxylé d'un oestrogène non stéroïdique phénolique choisi dans le groupe constitué par benzestrol, dinestrol, diéthylstilbestrol, hexestrol mestilbol et promethestrol;

(e) le résidu monodéshydroxylé d'un phénol bio-affectant choisi dans le groupe constitué par vitamine $K^5$, 4-acétamino-2-méthyl-1-naphtol, juglone, α-tocophérol, β-tocophérol, γ-tocophérol et δ-tocophérol;

(f) le résidu mono- ou polydéshydroxylé d'un analgésique narcotique ou antagoniste narcotique phénolique choisi dans le groupe constitué par apomorphine, hydromorphone, cétobémidone, morphine, nalorphine, naloxone, pentazocine, levorphanol, métopon, buprénorphine, butorphanol, cycloazocine, profadol, levallorphan, alazocine, nalbufine, oxilorphan, nalmexone, naltrexone et apocodéine, cyclorphan, désomorphine, dihydromorphine, 3-hydroxy-N-méthyl-morphinane, hydroxypéthidine, levophénacyl-morphan, métazocine, norlevophanol, normorphine, oxymorphone, phénazocine, phénomorphan, pholcodine et cyprenorphine;

(g) le résidu monodéshydroxylé d'un phénol bio-affectant choisi dans le groupe constitué par chlorquinaldol, 5-chloro-8-quinolinol, clamoxyquine, acide 8-hydroxy-7-iodo-5-quinoléinesulfonique, iodochlorohydroxyquine, octoquine, oxyquinoléine, 8-hydroxy-quinoléine, chloroxine amodioquine, diiodohydroxyquine et nitroxoline.

8. Procédé tel que défini dans la revendication 1 pour préparer un composé tel que défini dans la revendication 1, où $R^2$ est choisi dans le groupe constitué par alcoyle et aryle, et $R^3$ est choisi dans le groupe constitué par hydrogène, alcoyle et aryle.

9. Procéde tel que défini dans la revendication 1 pour préparer un composé tel que défini par la revendication 1, qui le N-phénoxy-méthylbenzamide, N-(4-acétamidophénoxyméthyl)benzamide, α-(N-méthyl)aminomethyl-3-(pivaloyloxymethoxy)-benzyl-alcool, α-pivaloyloxyanisol, 3,4, bis(pivaloy-loxyméthy)benzaldéhyde, 3-pivaloyloxy-4-pivaloyloxy-méthoxybenzaldéhyde.